# EUROPEAN PATENT APPLICATION

(11) **EP 4 272 625 A1**
(43) Date of publication of application: **08.11.2023**
(21) Application number: 21914598.4
(22) Date of filing: 30.12.2021
(51) Int. Cl.: A61B 5/00

(54) **INTERFACE DISPLAY METHOD, AND MONITOR AND COMPUTER STORAGE MEDIUM**

(30) Priority: 30.12.2020 WO PCT/CN2020/141347
(71) Applicant: Shenzhen Mindray Bio-Medical Electronics Co., Ltd., Shenzhen, Guangdong 518057 (CN)
(72) Inventor: HE, Zhilu, Shenzhen, Guangdong 518057 (CN); CAO, Jianfang, Shenzhen, Guangdong 518057 (CN); JIANG, Xia, Shenzhen, Guangdong 518057 (CN); YUAN, Weiwei, Shenzhen, Guangdong 518057 (CN); QING, Lei, Shenzhen, Guangdong 518057 (CN)
(74) Representative: KIPA AB
(86) International application number: PCT/CN2021/142872
(87) International publication number: WO 2022/143867

(57) **Abstract**

An interface display method, and a monitor and a computer storage medium, which are used for providing parameter layout pages corresponding to a plurality of measurement modes, such that a user selects a target parameter layout page suitable for an actual use situation. The method comprises: a user being able to select a target measurement mode according to a usage scenario of a monitor or a working habit of the user; and after a measurement mode selection instruction is input to the monitor, the monitor determining, on the basis of the measurement mode selection instruction, a target parameter layout page that needs to be displayed by the user, and presenting the target parameter layout page to the user. Therefore, a monitor can display different parameter layout pages according to different usage scenarios or working habits of users, thereby meeting different measurement requirements of users, such that the monitor can adapt to a usage situation of users.

## Description

### CROSS-REFERENCE TO RELATED APPLICATIONS

This disclosure claims priority to PCT application No. PCT/CN2020/141347, filed on December 30, 2020, to International Patent Office, titled "INTERFACE DISPLAY METHOD, MONITOR AND COMPUTER STORAGE MEDIUM"; the contents of which are incorporated herein by reference in its entirety.

### TECHNICAL FIELD

The disclosure relates to the technical field of medical device, and more particularly to an interface display method, and a monitor and a computer storage medium.

### BACKGROUND

Medical institutions, such as General Wards, Emergency Rooms, Doctor Clinics and Nursing homes, often measure vital signs of patients regularly, such as measuring parameters related to vital signs, such as temperature, blood pressure, pulse rate or respiratory rate and etc., and use monitors to display and record various parameters.

In the actual use of the monitor, the parameters, that need to be displayed and recorded by the user, vary depending on the situations. For example, in some departments, medical staff may need the monitor to display parameters, such as body temperature, single blood pressure, pulse rate and respiratory rate, while in other departments, medical staff may also need the monitor to display average blood pressure, or height, weight and other indicators, in addition to temperature, single blood pressure, pulse rate and respiratory rate. Therefore, the parameters displayed by the monitor vary depending on the situations in which it is located.

The types of parameters displayed on the display interface of existing monitor are fixed and can be applied to one specific situation. However, due to the different requirements for the parameter display of the monitor in other situations, such as the requirement to display more parameters or other types of parameters in other situations, the monitor that is suitable for the specific situation is no longer suitable for other situations, making it difficult for the monitor to be used in multiple situations.

### SUMMARY

The embodiment of this disclosure provides an interface display method, a monitor, and a computer storage medium for providing parameter layout pages corresponding to multiple measurement modes, enabling user to select target parameter layout pages suitable for actual usage situations.

A first aspect of an embodiment of this disclosure provides an interface display method which is applied to a monitor, including:
displaying a work interface on a display screen through the monitor;
displaying measurement modes in the work interface, wherein each measurement mode is associated with a corresponding parameter layout page; the measurement modes are at least configured to characterize a working mode of the monitor, wherein the working mode includes a continuous measurement working mode and a discontinuous measurement working mode; determining multiple first measurement modes according to the continuous measurement working mode, wherein the multiple first measurement modes respectively correspond to multiple first parameter layout pages; determining multiple second measurement modes according to the discontinuous measurement working mode, wherein the multiple second measurement modes respectively correspond to multiple second parameter layout pages; wherein the first parameter layout pages are at least partially different from the second parameter layout pages;
receiving a measurement mode selection instruction, which is configured to select a target measurement mode from a measurement mode identifier list, wherein the measurement mode identifier list includes the multiple first measurement modes and the multiple second measurement modes; and
in response to the measurement mode selection instruction, displaying a target parameter layout page which corresponds to the target measurement mode.
A second aspect of an embodiment of this disclosure provides a monitor, including a human-machine interaction module and a processor;
the human-machine interaction module is configured to receive a measurement mode selection instruction and/or a selection instruction for a functional control element, which are/is inputted by a user, wherein the human-machine interaction module is further configured to display a work interface;
wherein the work interface includes:
   a mode selection area, which is configured to display measurement modes and to receive the measurement mode selection instruction, wherein each measurement mode is associated with a corresponding parameter layout page; the measurement modes are at least configured to characterize a working mode of the monitor, wherein the working mode includes a continuous measurement working mode and a discontinuous measurement working mode; wherein the mode selection area is further configured to determine multiple first measurement modes according to the continuous measurement working mode, wherein the multiple first measurement modes respectively correspond to multiple first parameter layout pages; wherein the mode selection area is further configured to determine multiple second measurement modes according to the discontinuous measurement working mode, wherein the multiple second measurement modes respectively correspond to multiple second parameter layout pages; wherein the first parameter layout pages are at least partially different from the second parameter layout pages; wherein the measurement mode selection instruction is configured to select a target measurement mode from a measurement mode identifier list, wherein the measurement mode identifier list includes the multiple first measurement modes and the multiple second measurement modes;
   a state display area, which is configured to display state information of the monitor;
   a parameter report area, which is configured to display a target parameter layout page which corresponds to the target measurement mode, according to the target measurement mode; wherein the target parameter layout page includes at least a display area of a measurement parameter and measurement data which is located within said display area; wherein when the target parameter layout page belongs to the first parameter layout pages, the target parameter layout page includes a first parameter report area; when the target parameter layout page belongs to the second parameter layout pages, the target parameter layout page includes a second parameter report area, wherein the first parameter report area is at least partially different from the second parameter report area;
   a functional control element area, which includes at least one functional control element;
   wherein the processor is configured to execute a processing flow which corresponds to a selected target functional control element, in response to the selection instruction for the functional control element.

A third aspect of an embodiment of this disclosure provides a computer storage medium, in which instructions are stored; where when instructions are executed on a computer, the computer executes the method of the first aspect mentioned above.

From the above technical solutions, it can be seen that the embodiments of this disclosure have the following advantages.

In the embodiments of this disclosure, users can select a target measurement mode according to usage situations of the monitor or their own work habits. After inputting a measurement mode selection instruction to the monitor, the monitor determines a target parameter layout page that the users need to display according to the measurement mode selection instruction, and displays the target parameter layout page to the users. Therefore, the monitor can display different parameter layout pages according to different usage situations or user work habits, satisfying different measurement requirements of users, making the monitor being able to adapt to usage situations of users.

### BRIEF DESCRIPTION OF THE DRAWINGS

FIG. 1 is a schematic diagram of an application situation of a monitor in an embodiment of this disclosure.
FIG. 2 is a flowchart of an interface display method in an embodiment of this disclosure.
FIG. 3 is another flowchart of a display method in an embodiment of this disclosure.
FIG. 4 is a schematic diagram of a method for doing layout of several measurement parameters on an editing page according to parameter layout information in an embodiment of this disclosure.
FIG. 5 is a schematic diagram of a method for changing a measurement parameter to a target parameter according to change information in an embodiment of this disclosure.
FIG. 6 is a schematic diagram of a method of changing a layout position of a measurement parameter on a page to be edited according to position change information in an embodiment of this disclosure.
FIG. 7 is a schematic diagram of a measurement mode for setting a measurement parameter according to measurement setting information in an embodiment of this disclosure.
FIG. 8 is a schematic diagram showing adding a measurement parameter to an editing page according to addition information in an embodiment of this disclosure.
FIG. 9 is a schematic diagram showing setting a parameter type for a measurement parameter according to type setting information in an embodiment of this disclosure.
FIG. 10 is a schematic diagram of a receiving method for a measurement mode selection instruction in an embodiment of this disclosure.
FIG. 11 is a schematic diagram showing switching parameter report pages in an embodiment of this disclosure.
FIG. 12 is a schematic diagram of a method for distinguishing displaying display areas according to a measurement order in an embodiment of this disclosure.
FIG. 13 is another flowchart of an interface display method in an embodiment of this disclosure.
FIGS. 14-a and 14-b are schematic diagrams of switching processes of measurement modes by a monitor, according to a measurement mode switching instruction in an embodiment of this disclosure.
FIG. 15 is a schematic diagram of switching processes of measurement modes by a monitor, according to a measurement mode switching instruction which is inputted by swiping operation of user, in an embodiment of this disclosure.
FIG. 16 is a schematic diagram of a process in which a monitor re-measures updated parameters according to a re-measurement instruction in an embodiment of this disclosure.
FIG. 17 is a schematic diagram of a process in which a monitor opens/hides a display area according to a display area operation instruction in an embodiment of this disclosure.
FIG. 18 is an interface display diagram of a monitor in a standby mode in an embodiment of this disclosure.
FIG. 19 is a schematic diagram of a parameter layout page which corresponds to one measurement mode in an embodiment of this disclosure.
FIG. 20 is a schematic diagram of a parameter layout page which corresponds to another measurement mode in an embodiment of this disclosure.
FIG. 21 is a schematic diagram of a parameter layout page which corresponds to another measurement mode in an embodiment of this disclosure.
FIG. 22 is a structural diagram of a monitor in an embodiment of this disclosure.
FIG. 23 is a schematic diagram of a working interface of a monitor in an embodiment of this disclosure.
FIG. 24 is another structural diagram of a monitor in an embodiment of this disclosure.
FIG. 25 is a schematic diagram of another working interface of a monitor in an embodiment of this disclosure.

### DESCRIPTION OF THE PREFERRED EMBODIMENTS

The embodiment of this disclosure provides an interface display method, a monitor, and a computer storage medium for providing parameter layout pages corresponding to multiple measurement modes, thus enabling users to select target parameter layout pages suitable for actual usage situations.

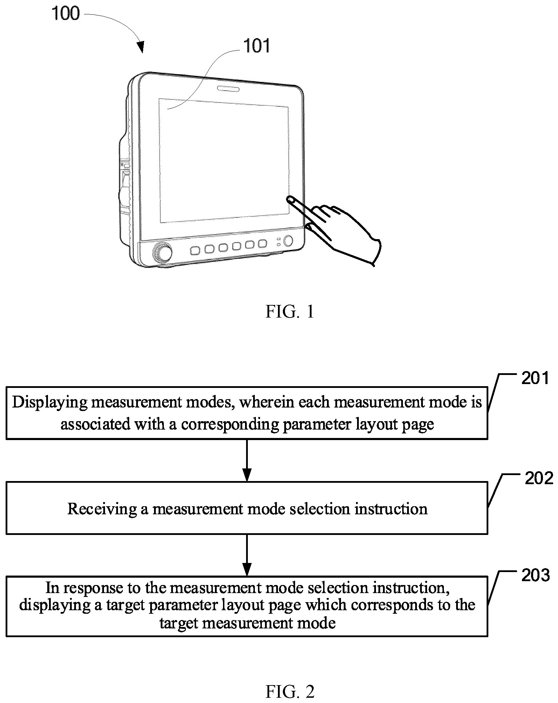

FIG. 1 is a schematic diagram of an application situation in an embodiment of this disclosure. Refer to FIG. 1, the application situation of an embodiment of this disclosure include a monitor 100.

The monitor 100 includes a human-machine interaction module 101 which is configured to display a work interface, a processor which is configured to process user instructions and/or execute preset computer programs, and a memory which is configured to store computer programs or data. Furthermore, the processor can be configured to communicate with the memory and execute a series of instruction operations corresponding to computer programs in the memory on the monitor 100.

In an embodiment of this disclosure, the working interface displayed by the human-machine interaction module 101 can be specifically configured to display information, such as physiological parameters and/or monitor state to users. The physiological parameters can be any physiological parameters, such as ECG data, respiration parameters, non-invasive blood pressure, oxygen saturation, pulse, body temperature, etc. The monitor state can specifically be information related to the monitor, such as electric quantity information, current time, working state, etc.

The user can input operation instructions to the monitor 100 through the human-machine interaction module 101 and receive data information displayed by the human-machine interaction module 101. In some embodiments, the human-machine interaction module 101 may be equipped with a display screen for displaying data information to the user. In one implementable method, the display screen is also a touch panel, which can be configured to receive gesture operation from the user on the touch panel, which can be further recognized as an operation instruction from the user. In another implementable method, the human-machine interaction module 101 can also be equipped with physical buttons (including knobs, buttons, etc.), which can be operated by the user to input the operation instruction to the monitor 100.

It can be understood that the human-machine interaction module 101 can be simultaneously equipped with the physical button and the display screen as the touch panel to receive operation instructions from the user. It is also possible for the human-machine interaction module 101 to only be equipped with the physical buttons, or to not be equipped with the physical buttons and only use the display screen as the touch panel, so as to receive operation instructions from the user. There are specific limitations herein.

In the embodiment of this disclosure, the monitor 100 can switch between different measurement modes according to different measurement situations (such as General Ward, Emergency Room, and other usage situations), or according to the operation instructions from the user. Each measurement mode can correspond to one parameter layout page, thereby satisfying the different measurement requirements of the user and highlighting the focuses of the user in different usage situations, so that the monitor can adapt to the usage situation of the user. Wherein, the measurement mode is determined according to the measurement situation and working mode. In some embodiments, the measurement situation is an application situation of the monitor 100, which can include a measurement region and/or a hospital working subarea. The hospital working subarea further includes: department type and public area. The department type further include: Emergency Department, General Ward, Ambulatory Surgery Center, etc. The working mode is a parameter measurement mode performed by the monitor 100, which can be divided according to continuity between at least two times of parameter measurement. In some embodiments, the working modes include continuous measurement working mode and spot check mode. In some embodiments, in the continuous measurement mode, the monitor 100 automatically obtains measurement results of at least two times of parameter measurement at a preset time interval. In spot check mode, there is no preset time interval between measurement results of at least two times of parameter measurement of the monitor 100, or the preset time interval is longer than that of the continuous measurement working mode.

In some embodiments, the measurement situation include a measurement region and a hospital working area division. Department types in FDA or US regions include: General Ward, Emergency Department, Doctor Office, Long Term Care, and ASC (Ambulatory surgery center). Department types in CE region or Europe region include: General Ward, Emergency Department, Physician Office, and ASC. The department types in China include: General Ward, Emergency Department, Community Health Service Center, ASC, and Neonatal Department. In practical applications, different department types can be displayed according to different regions as measurement situations for the user to select from. The measurement mode can be set to a default measurement mode at the factory. In one embodiment, the default measurement situation is a General Ward. In some embodiments, the default measurement mode can be modified, deleted, edited, or saved. In some embodiments, when switching the hospital working subarea, the measurement mode set under that zone is automatically cleared.

In some embodiments, the measurement situation includes a measurement region, which includes at least one of the following: the United States, Europe, and China. In some embodiments, the measurement situation includes a hospital working subarea, which at least includes: department type.

In some embodiments, the department type includes at least General Ward and Emergency Department. When the measurement situation is General Ward, the continuous measurement working mode includes a continuous monitoring mode, the discontinuous measurement working mode includes a general round mode and an initial evaluation mode. In some embodiments, the parameter layout page of the initial evaluation mode includes an orthostatic hypotension evaluation tool. When the measurement situation is emergency department, the continuous measurement working mode includes a continuous monitoring mode, the discontinuous measurement working mode includes a triage mode and a spot check mode.

The continuous monitoring mode refers to continuous measurement of vital sign parameters of a patient over a period of time. In this mode, abnormal changes in vital signs of the patient can be monitored and an alarm notification can be given. The spot check mode refers to single measurement of the vital sign parameters of the patient, in which the single measured vital sign parameter can be stored and further transmitted to other devices. The general round mode refers to spot-checking the vital sign parameters of multiple patients in sequence, and in this mode, the vital sign parameter of a single patient can be transmitted to other devices. The initial evaluation mode refers to a single measurement of vital sign parameter for the patient for obtaining an initial evaluation of physical condition according to the measurement result, such as an initial evaluation for risk of falls. The triage mode refers to a single measurement of vital sign parameter for the patient to assess critical degree or level of the patient, so as to determine department to be visited by the patient.

It should be noted that the specific implementation of the same working mode for different monitoring situations can be set according to the requirement of each monitoring situation, and may not be completely the same.

The department type further includes one of followings: Doctor Office, Long Term Care, ASC, Community Health Service Center, and Neonatal Department.

When the department type is Doctor Office or Community Health Service Center, the continuous measurement working mode includes a continuous monitoring mode, while the discontinuous measurement working mode includes a spot check mode. In some embodiments, when the department type is Doctor Office or Community Health Service Center, the parameter layout page in the spot check mode includes a height display area, a weight display area, and an average blood pressure tool.

When the department type is Long Term Care, the continuous measurement working mode includes a continuous monitoring mode, while the discontinuous measurement working mode includes a general round mode and an initial evaluation mode. Due to the similarity between the application situations of Long Term Care and General Ward, the only difference between them is inside or outside hospital. Therefore, the working mode settings of the two are also similar.

When the department type is ASC, the continuous measurement working mode includes a continuous monitoring mode, while the discontinuous measurement working mode includes a recovery mode. The recovery mode refers to a single measurement of vital sign parameter for the patient before or after surgery, so as to evaluate or confirm patient condition before or after surgery. In some embodiments, the non-invasive blood pressure measurement in the recovery mode is preset with a preset time interval between two times of parameter measurement, as well as preset with a preset measurement duration and/or preset measurement frequency of the measurement implemented according to the preset time interval. In some embodiments, the parameter layout page of the recovery mode includes a parameter display area of carbon dioxide.

When the department type is Neonatal Department, the continuous measurement working mode includes a continuous monitoring mode, while the discontinuous measurement working mode includes a spot check mode. In some embodiments, when the department type is Neonatal Department, the parameter layout page includes a CCHD (Cyanotic Congenital Heart Disease) monitoring tool, a blood oxygen display area, and a pulse display area.

The following is a description of an interface display method in an embodiment of this disclosure, combined with the application situation shown in FIG. 1.

Please refer to FIG. 2. One embodiment of the interface display method in an embodiment of this disclosure includes followings.

The interface display method of this embodiment is applied to the monitor, which can display a work interface on a display screen of the monitor. The work interface displayed on the display screen by the monitor can be configured to perform the following multiple steps.

In step 201, measurement modes are displayed, wherein each measurement mode is associated with a corresponding parameter layout page. The monitor displays a work interface on the display screen, which is configured to display the measurement modes. Each measurement mode is associated with a corresponding parameter layout page. The monitor can be configured to measure physiological parameters of a subject and display measurement results of different types of measurement parameters in the work interface. In a specific measurement mode, the work interface displays various measurement parameters which correspond to that measurement mode. These various measurement parameters are respectively arranged in different areas and positions on a page, which is displayed on the work interface, forming a parameter layout page which corresponds to these various measurement parameters. That is to say, each measurement mode corresponds to one parameter layout page. The measurement mode is configured to characterize the working mode of the monitor, which includes a continuous measurement working mode and a discontinuous measurement working mode.

Wherein, multiple parameter layout pages, which correspond to multiple measurement modes, can be the same or different, and there is no limitation for this herein.

In some embodiments, multiple first measurement modes can be determined according to the continuous measurement working mode, wherein the multiple first measurement modes respectively correspond to multiple first parameter layout pages. Multiple second measurement modes can be determined according to the discontinuous measurement working mode, wherein the multiple second measurement modes respectively correspond to multiple second parameter layout pages. The first parameter layout pages are at least partially different from the second parameter layout pages.

In this embodiment, the monitor can obtain the parameter layout page which corresponds to each measurement mode, so as to display the corresponding parameter layout page to the user when the user selects a certain measurement mode.

In step 202, a measurement mode selection instruction is received.

When it is necessary to measure and record parameters according to a certain measurement mode, the measurement mode selection instruction, which is configured to select a target measurement mode from the measurement mode identifier list, is inputted. The measurement mode identifier list includes multiple first measurement modes corresponding to the continuous measurement working mode and multiple second measurement modes corresponding to the discontinuous measurement working mode.

In step 203, a target parameter layout page, which corresponds to the target measurement mode, is received, in respond to the measurement mode selection instruction.

After receiving the input measurement mode selection instruction, the monitor responds to the measurement mode selection instruction, so as to determine the target parameter layout page which corresponds to the target measurement mode from multiple parameter layout pages.

After determining the target parameter layout page, the monitor displays the target parameter layout page through the work interface, so that the user can execute the corresponding target measurement mode according to the target parameter layout page, and complete the measurement and record of measurement parameters in this target measurement mode. In some embodiments, the target parameter layout page is displayed in a parameter report area of the work interface.

In this embodiment, the monitor has multiple working modes, wherein each working mode corresponds to multiple measurement modes, and each measurement mode has a corresponding parameter layout page. Therefore, the user can select the target measurement mode according to different conditions, such as usage situations of the monitor, monitoring requirements of patient, or work habits of the user. After inputting the measurement mode selection instruction to the monitor, The monitor determines the target parameter layout page that the user needs to display, according to the measurement mode selection instruction, and displays the target parameter layout page to the user. Therefore, the monitor can display different parameter layout pages according to different usage situations of the monitor or different work habits of users, satisfying the different measurement requirements of user and enabling the monitor to adapt to their usage situations.

In the embodiment of this disclosure, there are multiple manners to acquire the parameter layout page which corresponds to each measurement mode. In one acquisition manner, the parameter layout page can be customized through the monitor, so that the monitor can acquire the customized parameter layout page. In another acquisition manner, the configured parameter layout page can be imported to the monitor, so that the monitor receives the imported parameter layout page. According to the embodiment shown in FIG. 2, the above two manners are elaborated in detail below.

In some embodiments, the configuration parameter layout page can be customized.

In this embodiment, customized configuration and editing are performed on an editing page, and the parameter layout page can be obtained after completing the customized configuration and editing.

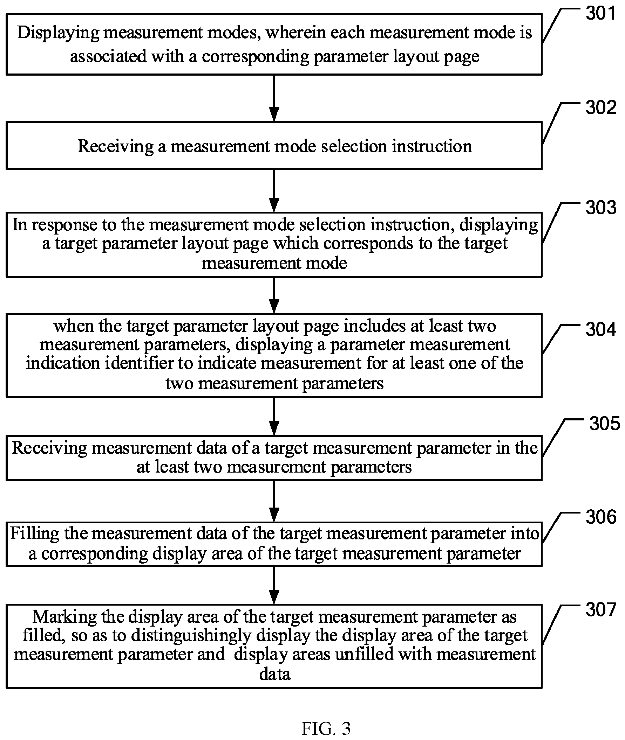

Please refer to FIG. 3. Another embodiment of an interface display method in an embodiment of this disclosure includes following steps.

In step 301, measurement modes are displayed, wherein each measurement mode is associated with a corresponding parameter layout page.

In this embodiment, the acquisition manner of the parameter layout page can be as follows. The monitor provides an editing page for the parameter layout page, and editing information can be inputted on this editing page. This editing information represents relevant operation information for the measurement parameter on the editing page. Therefore, the monitor receives this editing information and operates the measurement parameter on the editing page according to this editing information. The operation performed on the measurement parameter is the editing of the editing page. After the editing is completed, the monitor generates a parameter layout page according to the editing information of the editing page.

Specifically, in a specific embodiment, the editing manner for the editing page can be as follows. The editing information includes parameter layout information, which is configured to represent layout information of several measurement parameters on the editing page. The layout information can specifically include information such as layout positions of the measurement parameters and size of layout area. Therefore, after receiving the parameter layout information, several measurement parameters can be laid out on the editing page according to the parameter layout information.

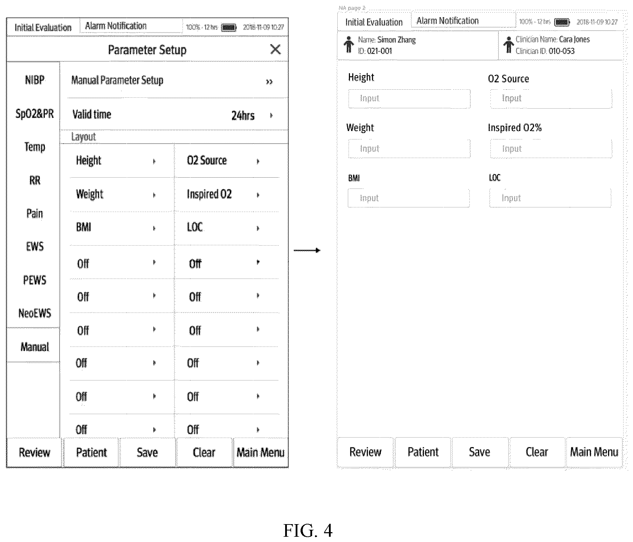

FIG. 4 is a schematic diagram of a method for doing layout of several measurement parameters on an editing page according to parameter layout information. The figure on the left side of the arrow is the editing page, and the figure on the right side of the arrow is the parameter layout page generated by the monitor on the editing page, according to the parameter layout information. As shown in the figures, the monitor displays the editing page. In the "Layout" area of the editing page, multiple measurement parameters, such as "Height" height parameter and the "Weight" weight parameter, can be laid out by the user. By inputting the parameter layout information to the monitor, which parameter layout information indicates that the layout position of the "Height" height parameter is directly above the layout position of the "Weight" weight parameter, and the layout position of BMI index is directly below the layout position of the "Weight" weight parameter, etc. Therefore, after receiving the parameter layout information, the monitor executes the corresponding layout according to the parameter layout information. After completing the parameter layout, the parameter layout page can be generated.

This embodiment of the monitor provides a parameter layout method, in which the user can place some parameters, that need to be focused on, in prominent positions, on the page for easy viewing. The users can adjust the parameter layout according to their own usage habits and other factors, making the interface configuration of the monitor more practical.

In another specific embodiment, the editing manner of the editing page can also be as follows. The editing information includes change information of the measurement parameter, which change information is configured to represent that the measurement parameter in the editing page are changed to a target parameter. Therefore, after receiving the change information for the measurement parameter, the monitor changes the measurement parameter to the target parameter indicated by the change information, according to the change information.

Among them, there are various manners to receive the change information. In one manner, the monitor displays an optional parameter list, and the target parameter is selected from multiple optional parameters in the list to replace the measurement parameter on the editing page. This is equivalent to inputting change information to the monitor, and the monitor can receive the change information.

There are other manners to receive the change information, and there are no restrictions for these herein. For example, the monitor can display an input box to the user, where the user can input parameter name of the target parameter, and then the monitor can determine to change the measurement parameter in the editing page to the target parameter corresponding to the parameter name inputted by the user.

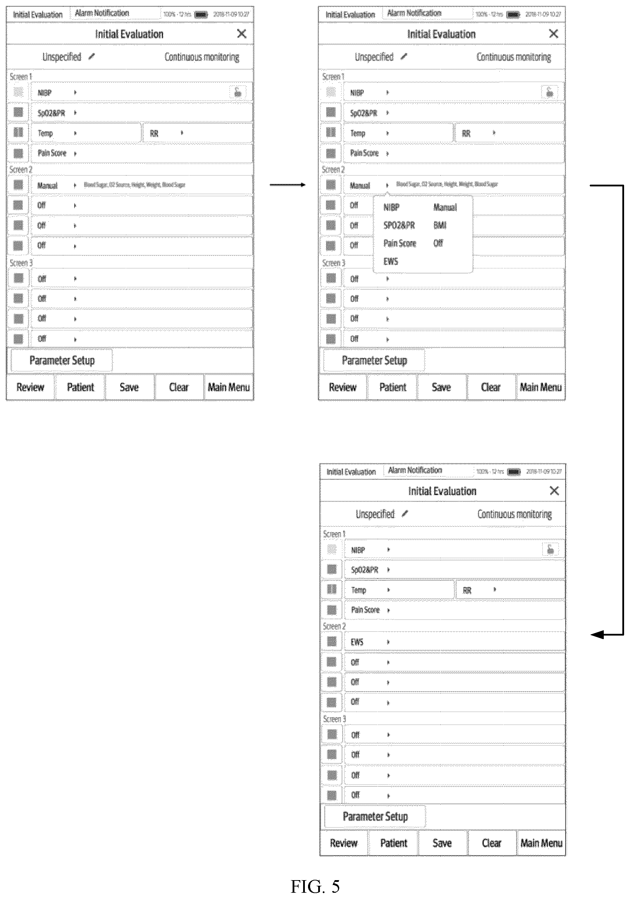

FIG. 5 is a schematic diagram of a method for changing a measurement parameter to a target parameter according to change information in an embodiment of this disclosure. As shown in the figure, if the user needs to change the measurement parameter "Manual (parameter manually measured)", the user can click on a triangle symbol near the "Manual" parameter, and the monitor receives click operation of the user on the triangle symbol, so as to display the optional parameter list to the user. The user can select the target parameter from multiple optional parameters in the optional parameter list. For example, if the user selects the "EWS (early warning score)" parameter, the monitor responds to the selection operation of the user for the "EWS" parameter by changing the "Manual" parameter to the "EWS" parameter.

In the above method, the user can change parameters according to the actual situation, making the measurement mode of the monitor more suitable for the current situation, and enabling the page design of the monitor more personalized and adaptable to the actual requirements of users.

In another specific embodiment, the editing manner of the editing page can also be as follows. The editing information inputted by the user on the editing page includes a position change information of the measurement parameter on the editing page, which position change information is configured to represent a change of a layout position of the measurement parameter on the editing page. Therefore, after receiving the position change information of the measurement parameter, the monitor changes the layout position of the measurement parameter on the editing page according to the position change information.

Among them, the method of receiving the position change information of the measurement parameters can be as follows. If the user drags the measurement parameter through an input gesture, the monitor receives gesture dragging operation inputted by the user, which represents moving the measurement parameter to the target position. Therefore, the monitor responds to the gesture dragging operation, so as to move the measurement parameter from the current layout position to the target position.

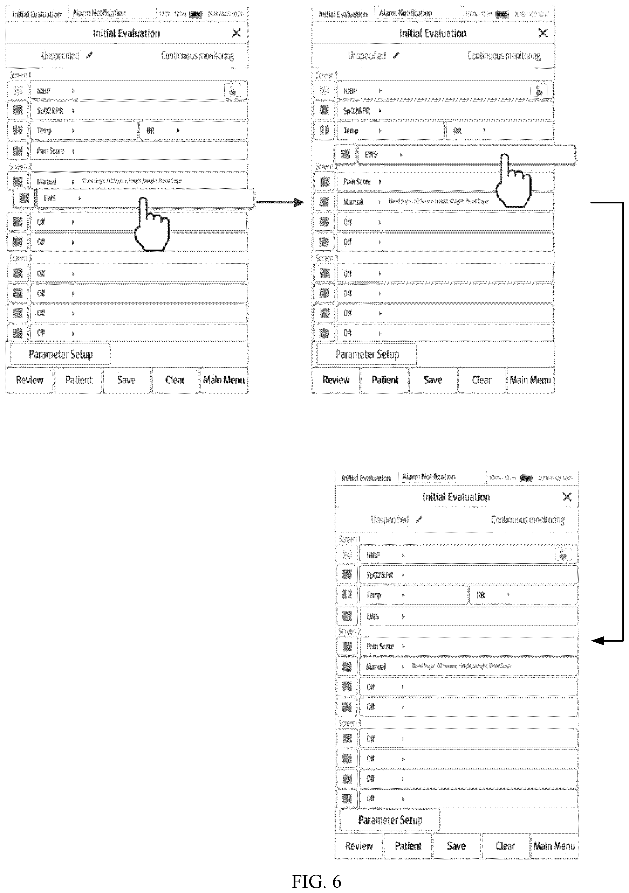

FIG. 6 is a schematic diagram of a method of changing a layout position of a measurement parameter on a page to be edited according to position change information. As shown in the figure, the user needs to change the layout position of the measurement parameter "EWS" and move it from "screen 2 (second screen) " to "screen 1 (first screen)" for easy viewing of "EWS" parameters. For this purpose, the user drags the "EWS" parameter to the layout position of the "Pain Score" parameter through gesture, and the monitor receives the gesture dragging operation inputted by the user, and moves the layout position of the "EWS" parameter from "screen 2" to the layout position where the "Pain Score" parameter is located in "screen 1". In some embodiments, the first screen includes at least one target parameter. In some embodiments, the first parameter report area includes at least one target parameter.

In another specific embodiment, the editing manner of the editing page can also be as follows. The editing information inputted by the user on the editing page includes measurement setting information of the measurement parameter, which is configured to set a measurement mode of the measurement parameter. Therefore, the monitor sets the measurement mode of the measurement parameter to a corresponding measurement mode according to the measurement setting information.

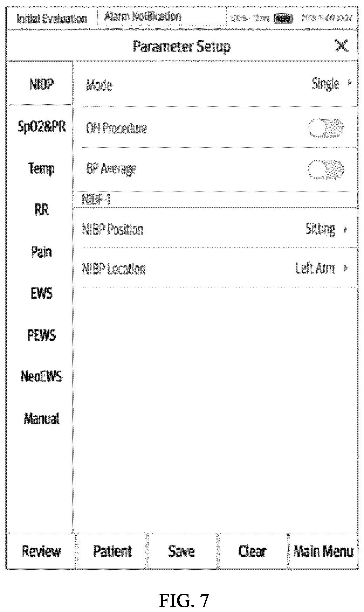

FIG. 7 is a schematic diagram of a measurement mode for setting a measurement parameter according to measurement setting information. As shown in the figure, the user needs to set the measurement mode for NIBP parameter (automated non-invasive blood pressure). The monitor provides the user with multiple measurement modes for NIBP parameter, such as OH Procedure (orthostatic hypotension measurement), BP Average (average blood pressure), measurement portion of NIBP parameter, and posture of NIBP subject (standing, sitting, or lying), the user can input the measurement setting information according to the various measurement modes provided by the monitor. For example, the measurement setting information indicates that the measurement mode of NIBP parameters is to measure the left arm blood pressure of the subject during sitting, and the monitor sets the measurement mode of NIBP parameters according to this measurement setting information to measure the left arm blood pressure of the subject during sitting.

In another specific embodiment, the editing manner of the editing page can also be as follows. The editing information inputted by the user on the editing page includes addition information of the measurement parameter, which is configured to add the measurement parameter on the editing page. Therefore, the monitor adds a measurement parameter to the editing page according to the addition information.

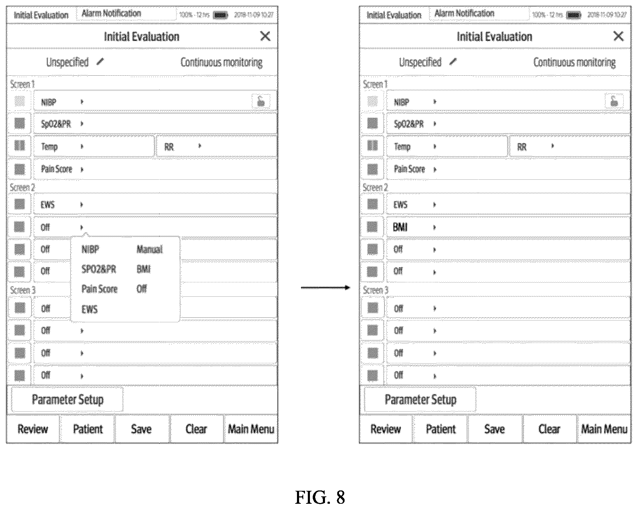

FIG. 8 is a schematic diagram showing adding a measurement parameter to an editing page according to addition information.

As shown in the figure, on the page of "screen 2", "off" indicates that no parameter is laid out at the current position. The user can add the parameter here. If the user clicks on the triangle symbol near "off", the monitor displays a parameter list to the user. The user selects the parameter to be added in the list, that is, input the addition information of the measurement parameter. For example, when selecting "BMI index", the monitor adds the "BMI index" to the page of "screen 2" according to the addition information.

In another specific embodiment, the editing manner of the editing page can also be as follows. The measurement parameter include a parameter with manually inputted measurement data, and the editing information inputted by the user on the editing page includes a type setting information of the measurement parameter, which type setting information is configured to set a parameter type of the measurement parameter. The parameter type includes numeric type and optional type. Therefore, the monitor sets the parameter type of the measurement parameter to the parameter type corresponding to the type setting information, according to the type setting information of the measurement parameter.

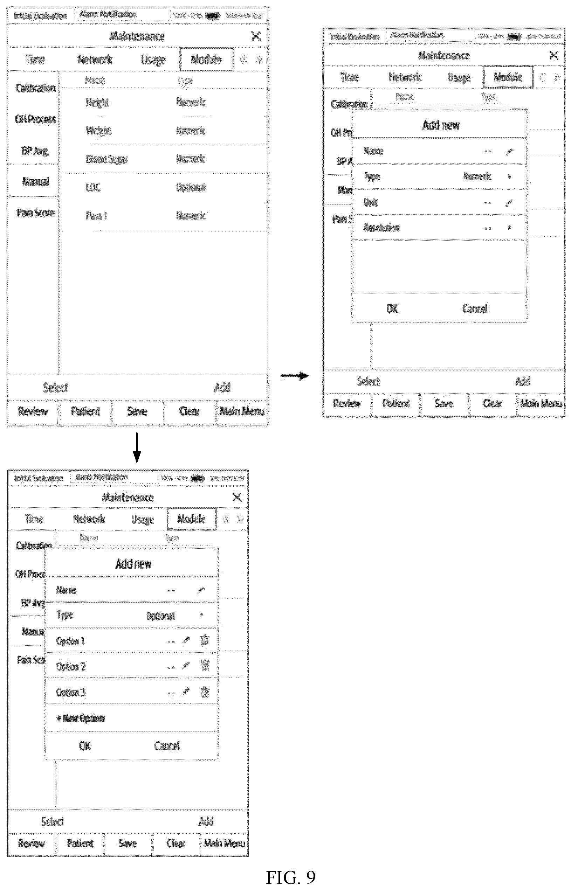

Wherein, the parameter with manually inputted measurement data refers to a parameter that requires the user to manually input measurement data according to an actual measurement result. For example, parameters, such as height and weight, need to be measured by the user, and the user should manually input the measurement data to the monitor, after obtaining the measurement results. Due to the fact that the parameter type can be either numeric or optional, the monitor can set the parameter type of the measurement parameter to numeric type or optional type, according to the type setting information inputted by the user. For example, FIG. 9 is a schematic diagram showing setting a parameter type for a measurement parameter according to type setting information. As shown in the figure, the user can click "Add" button to add a parameter and select name of the parameter to be added and set its parameter type in a pop-up dialog box. In the "Type" line, the user can select "Numeric" or "Optional", and selecting "Numeric" means that the user sets the parameter type to numeric, while selecting 'Optional' means setting the parameter type to optional.

When the measurement parameter is set as the numeric type, the user can also continue to set some details of the measurement parameter, that is, the user inputs the detailed setting information of the measurement parameter, which detailed setting information is configured to set parameter detail of the measurement parameter. The parameter detail includes one or more items of numeric unit, numeric input range, and numeric accuracy. The monitor sets the parameter detail of the measurement parameter to the corresponding parameter detail according to the detailed setting information.

When the parameter is set as the numeric parameter, the monitor can provide the user with an input box for a parameter value, and the user can manually input a specific value into this input box according to the actual measurement result. Moreover, after completing the parameter detail setting of the measurement parameter, the monitor displays the specific value of the parameter according to the set parameter detail.

For example, for the "height" parameter, the user can set it as a numeric parameter and further set the numeric unit (meters, centimeters, etc.) or numeric accuracy (such as precision to two decimal places) of the parameter. As shown in FIG. 9, the user can set the numeric unit of the "Height" parameter in the "Unit" line.

If the measurement parameter is set as an optional parameter, the monitor can set multiple options related to measurement data of the measurement parameter. For example, as shown in FIG. 9, the user can set the parameter type of the "LOC" parameter (level of consciousness) to optional type and provide multiple options such as "Option 1", "Option 2", and "Option 3". After setting the options, the monitor displays multiple options to the user when displaying the measurement data of the parameters. The user can select one option that matches the actual measurement result.

The above describes various editing manners for editing page. After executing each editing manner, the monitor can display an effect preview page of the edited editing page to the user. For example, after executing the parameter layout of the editing page or the parameter changing of the editing page, the monitor can display the effect preview page of the parameter layout of the editing page or the effect preview page of the parameter change to the user, enabling the user to predict the editing effect of the page in advance.

After the editing of the editing page is completed, a parameter layout page can be generated, with each parameter layout page corresponding to one measurement mode. The user can select one measurement mode according to actual measurement requirement for measurement. The monitor also displays the corresponding parameter layout page according to the measurement mode selected by the user. Among them, the parameter layout page edited by the user can be saved and easily accessed at any time when displaying the parameter layout page.

In step 302, a measurement mode selection instruction is received.

When the user uses the monitor, the monitor allows the user to select one measurement mode, that is, the monitor provides multiple measurement modes, and the user can input a measurement mode selection instruction, which represents selecting a target measurement mode from the multiple measurement modes.

Specifically, one manner to receive the measurement mode selection instruction can be as follows. The monitor display a measurement mode identifier list for the user. The measurement mode identifier list includes an identifier of each measurement mode, and the identifier includes a name of measurement situation. Selecting a target identifier on the list means that the user selects the corresponding target measurement mode for the target identifier, so that the monitor can determine the target measurement mode that the user needs to use.

Among them, the identifier of measurement mode can be name, code name, or graphic symbol of the measurement mode, as long as it can refer to a unique measurement mode, and its form is not limited.

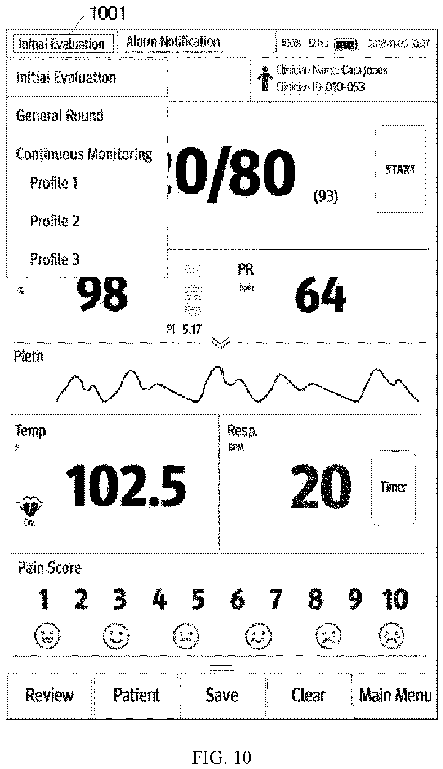

FIG. 10 is a schematic diagram of a receiving method for a measurement mode selection instruction. As shown in the figure, the user can click on button 1001, and the monitor displays a measurement mode identifier list for the user. This list includes multiple measurement mode identifiers, such as "Initial Evaluation", "General Round", and "Continuous Monitoring". Assuming that the target identifier selected by the user is "Initial Evaluation", the monitor can determine that the target measurement mode that the user needs to use is the measurement mode corresponding to "Initial Evaluation".

In addition, another manner to receive the measurement mode selection instruction can be as follows. The monitor receives a measurement mode selection instruction inputted by the user through swiping gesture in the currently displayed parameter layout page. This measurement mode selection instruction represents that the user selects the target parameter layout page from multiple parameter layout pages. For example, if a user swipes the currently displayed parameter layout page through swiping gesture, the currently displayed parameter layout page is switched to the next parameter layout page, wherein the swiping gesture can be in various directions such as up and down or left and right. Due to that each parameter layout page corresponds to one measurement mode, each swiping gesture of the user is perceived by the monitor as one measurement mode selection instruction inputted by the user, which measurement mode selection instruction represents the selection of the user for the target measurement mode. When using the swiping gesture to switch parameter layout pages and parameter report pages, the direction of swiping gesture can be defined to limit different switching objects. In some embodiments, the swiping gesture in a first direction is configured to switch parameter layout pages, while the swiping gesture in a second direction switches the work interfaces. In some embodiments, the first direction is defined as a length direction of the monitor 100, and the second direction is defined as a width direction of the monitor 100.

In this embodiment, there are various manners to the receive measurement mode selection instruction inputted by the user, and there are no specific limitations here. For example, this method can also be as follows. The monitor provides the user with an input box for a measurement mode identifier, and the user inputs the measurement mode identifier into this input box, so that the monitor determines the target measurement mode according to the inputted measurement mode identifier of the user.

In this embodiment, if the user believes that the currently displayed parameter layout page is not suitable for the current actual measurement situation, or believes that the currently displayed parameter layout page is not in line with usage habits of the user, the user can switch the currently displayed measurement mode, that is, the measurement mode selection instruction includes a measurement mode switching instruction inputted by the user, which instruction represents switching from the current measurement mode to the target measurement mode.

In step 303, a target parameter layout page which corresponds to the target measurement mode is displayed, in respond to the measurement mode selection instruction.

In this embodiment, if the measurement mode selection instruction includes a measurement mode switching instruction, the monitor responds to the measurement mode switching instruction and switches from the currently displayed parameter layout page to the target parameter layout page which corresponds to the measurement mode switching instruction.

If the measurement mode selection instruction is inputted by the user through the measurement mode identifier list, after determining the target measurement mode corresponding to the target identifier selected by the user, the target parameter layout page which corresponds to the target measurement mode is displayed to the user.

If the measurement mode selection instruction is inputted by the user through the swiping gesture, the target parameter layout page which corresponds to the measurement mode selection instruction is displayed to the user after each swiping gesture.

In this embodiment, each parameter layout page can be displayed through one or more parameter report pages, and each parameter report page includes one or more measurement parameters. When the parameter layout page is displayed through multiple parameter report pages, the multiple parameter report pages can be switched on the display.

The working interface of the monitor includes a parameter report area, which is configured to display the parameter report page of the parameter layout page. The parameter report area only displays one parameter report page, but multiple page parameter report pages can be switched and displayed separately in the parameter report area. The parameter report area includes a preset number of display lines. Therefore, when the number of display lines included in the parameter layout page exceeds the preset number of display lines in the parameter report area, the monitor displays the parameter layout page through at least two parameter report pages.

For example, the preset number of display lines in the parameter report area is 5, which means that the parameter report area can display up to 5 lines, with each line displaying at least one measurement parameter. When the number of display lines on the parameter layout page is 8, the first 5 lines of the parameter layout page serve as one parameter report page, and the last 3 lines of the parameter layout page serve as the next parameter report page. Therefore, the parameter layout page has two parameter report pages, and the parameter report area can switch to display any one of the two parameter report pages.

When the parameter layout page includes at least two parameter report pages, the monitor receives an instruction for selecting the parameter report page and selects a target parameter report page to display, according to this instruction.

Wherein, the preset number of display lines in the parameter report area can be any numeric value. Preferably, this preset number of display lines, is no more than 5. In addition, the parameter report area of the monitor also includes a preset number of display columns. Preferably, the preset number of display columns is less than or equal to 2 columns.

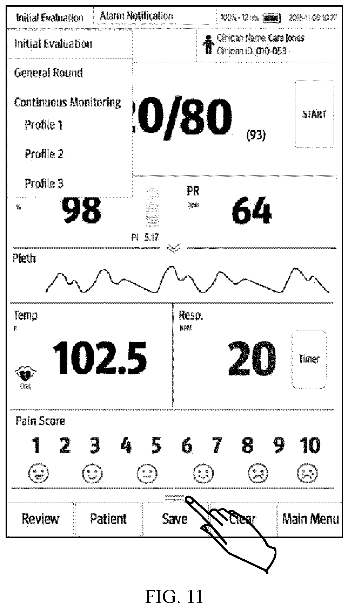

FIG. 11 is a schematic diagram showing switching parameter report pages. As shown in the figure, the user swipes the currently displayed parameter report page through gesture, and the monitor senses a selection instruction from the user for the parameter report page. According to this instruction, the currently displayed parameter report page is switched to the next parameter report page. Among them, the swiping gesture can be in various directions such as up, down, left, and right. It can be understood that in some embodiments, switching can also be achieved through other manners, such as clicking on a preset switching button or selecting through a rotary encoder, without being limited to the swiping gesture, as long as it can achieve the selection function.

In step 304, when the target parameter layout page includes at least two measurement parameters, a parameter measurement indication identifier is displayed to indicate measurement for at least one of the at least two measurement parameters.

After displaying the target parameter layout page, the user can use it to measure parameters. The target parameter layout page includes at least two measurement parameters. After obtaining the measurement result of the measurement parameter, the measurement result can be filled into the display area of the measurement parameter, making it easy for the user to view the measurement result.

In this embodiment, the monitor can provide a measurement indication for measurement parameter, that is, indicate a measurement parameter with a current measurement priority. Specifically, when the target parameter layout page includes at least two measurement parameters, the measurement parameters can be preset with a measurement order, and the measurement parameters are measured sequentially according to that measurement order. In some embodiments, parameter measurement indications for at least two measurement parameters are displayed sequentially on the target parameter layout page according to the measurement order.

After obtaining the measurement order of the measurement parameters, the monitor provides the measurement indication for at least one measurement parameter in the target parameter layout page in sequence according to the measurement order. In some embodiments, the target measurement parameter is determined among unmeasured measurement parameters according to the measurement order and the ranking position of the most recently measured measurement parameter in the measurement order.

Specifically, there are various display manners for the parameter measurement indication identifier. In some embodiments, the target measurement parameter can be displayed distinguishingly from the measurement parameters which are not the target measurement parameter in the target parameter layout page. The display area of the target measurement parameter can also be displayed distinguishingly from the display areas which are not the display area of the target measurement parameter on the target parameter layout page. The target measurement parameter is determined according to the measurement order, and the display area refers to the area where the measurement parameter is located. This display area can be specifically configured to display the measurement result of the measurement parameter, the name of the measurement parameter or the measurement mode of the measurement parameter. This embodiment does not limit the display manner of the parameter measurement indication identifier, as long as the parameter measurement indication identifier can indicate the measurement parameter with a current measurement priority.

Wherein, the distinguishing display manner can be distinguishingly displaying a border of the display area of the target measurement parameter and a border of other display areas. The distinguishing display of the border can be distinguishing display of colors of the borders or distinguishing display of line thicknesses of the borders.

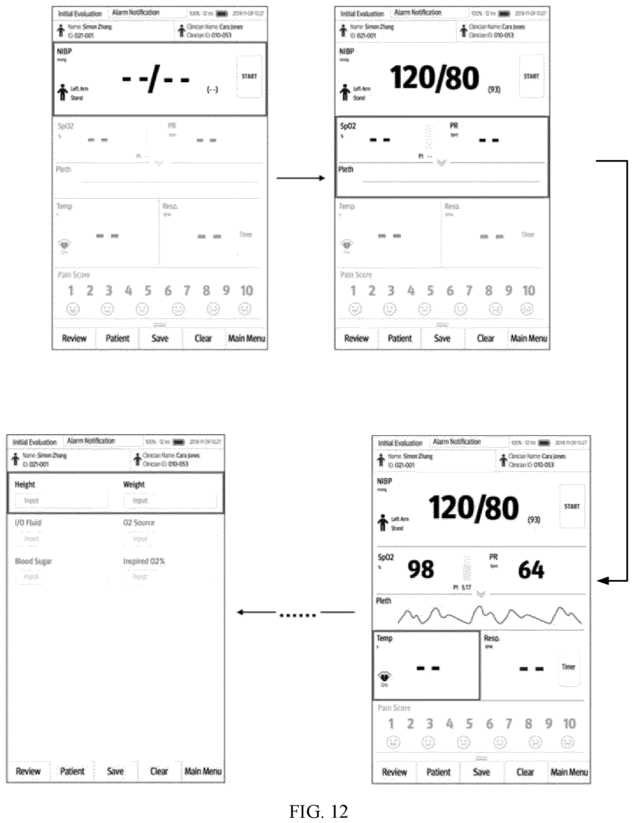

FIG. 12 is a schematic diagram of a method for distinguishing displaying display areas according to a measurement order. As shown in the figure, the measurement order of the measurement parameters in the target parameter layout page is NIBP parameter, SpO2 parameter (oxygen saturation), PR parameter (pulse rate), Pleth parameter (blood oxygen waveform), Temp parameter (body temperature).... Therefore, according to the measurement order, the target measurement parameter indicated by the first measurement is NIBP parameter, the target measurement parameters indicated by the second measurement are SpO2 parameter, PR parameter, and Pleth parameter... and so on. The measurement indications are sequentially displayed for the measurement parameters included in the target parameter layout page. During each measurement indication, the border of the display area of the target measurement parameter is displayed distinguishingly from that of other display areas, such as according to the line thickness of the border as shown in FIG. 12. That is, the line thickness of the border of the display area of the target measurement parameter is bold for each measurement indication. Priority measurement is given to the target measurement parameter during each measurement indication process, and the measurement result of the target measurement parameter is filled in the corresponding display area.

In addition, the measurement data, text, or background inside the display area of the target measurement parameter can also be displayed distinguishingly from the text or background in other display areas. This embodiment does not limit the specific method of distinguishing display, as long as it is able to distinguish the display area of the target measurement parameter from other display areas.

In this embodiment, in addition to distinguishing the display area, the specific method of measurement indication can also be as follows. The monitor pops up an indication box to indicate the user that the current target measurement parameter is A parameter. After completing the measurement of the A parameter, the monitor continues to pop up an indication box to indicate that the next target measurement parameter is B parameter, and so on, until all measurement parameters are indicated. This embodiment does not limit the specific manners of measurement indications.

This embodiment guides the medical staff to conduct parameter measurement in an orderly manner by providing measurement indications for measurement parameters. In cases where there are many measurement parameters or when the hospital is busy with work, the overall visual changes of the parameter layout page guide medical staff to avoid missing parameter measurement and ensure that parameter measurement are carried out in an orderly and accurate manner.

In some embodiments, the measurement order of parameters may not be set, but the measured and unmeasured measurement parameters can be distinguishingly displayed.

In step 305, measurement data of a target measurement parameter in the at least two measurement parameters, is received.

During the process of providing the measurement indication for the measurement parameter, the user can measure the target measurement parameter from the at least two of the measurement parameters included in the target parameter layout page, according to the measurement indication of the monitor, and obtain the measurement data of the target measurement parameter. The measurement data of the target measurement parameter can be obtained by measuring through an accessory probe of the monitor. For some parameters that cannot be measured by the monitor itself (such as height, weight, etc.), the user need to measure them and record the measurement data on the monitor. Regardless of the method used, the monitor can receive the measurement data of the target measurement parameter.

Among them, each measurement parameter has a corresponding display area on the target parameter layout page.

In step 306, the measurement data of the target measurement parameter is filled into a corresponding display area of the target measurement parameter.

After receiving the measurement data of the target measurement parameter, the measurement data is filled into the display area corresponding to the target measurement parameter, so that the measurement data can be displayed in the display area corresponding to the target measurement parameter, making it convenient for the user to view the measurement data.

In step 307, the display area of the target measurement parameter is marked as filled, so as to distinguishingly display the display area of the target measurement parameter and display areas which are unfilled with measurement data;
After filling in the measurement data of the target measurement parameter, the display area of the target measurement parameter is marked as filled, so that the display area of the target measurement parameter is distinguished from the display areas unfilled with measurement data, making it easy to visually see which measurement parameter is measured and which is not measured, thus avoiding the occurrence of missing measurement parameter.

Specifically, the specific manner for distinguishing display can be displaying a background of the display area of the target measurement parameter filled with measurement data in a color different from a background of the display area unfilled with the measurement data.

It can be understood that when setting the measurement order of parameters, the method of steps 306-308 can also be used for distinguishing display.

For example, as shown in FIG. 12, the display area with filled measurement data displays the measurement data, and the background of the display area (backgrounds for such as parameter name, measurement mode, etc.) is black, while the background of the display area unfilled with the measurement data is in a semitransparent gray state. By displaying the backgrounds of the display areas in different colors, it is possible to visually present the display areas filled and unfilled with measurement data, making it easy for the user to detect missing measurement parameters in a timely manner.

The specific manner of distinguishing display is not limited. For example, this method can also be to highlight the border of the display area filled with measurement data, while not highlight the border of the display area unfilled with measurement data. As long as the above two display areas are distinguished through visual differences, it is sufficient.

In this embodiment, in order to distinguish between the display area filled with measurement data and the display area unfilled with measurement data, in addition to distinguishingly displaying the two, the word "filled" can also be marked in the display area filled with measurement data and the word "unfilled" can be marked in the display area unfilled with measurement data. This embodiment does not limit the distinguishing method, as long as it is able to distinguish between the display area filled with measurement data and the display are unfilled with measurement data.

In this embodiment, the user can customize and edit the parameter layout page, making the parameter layout page more suitable for the actual use situation of the monitor and the own use habits of the user, and improving the realizability of the scheme.

In addition to the customized configuration and editing of parameter layout page described above, the monitor can also obtain the parameter layout page by importing the configured parameter layout page to the monitor, so that the monitor can receive the imported parameter layout page. On the basis of the embodiments shown in FIGS. 2 and 3, another method for obtaining parameter layout page is further elaborated in detail below.

In some embodiments, the configured parameter layout page can be directly imported into the monitor.

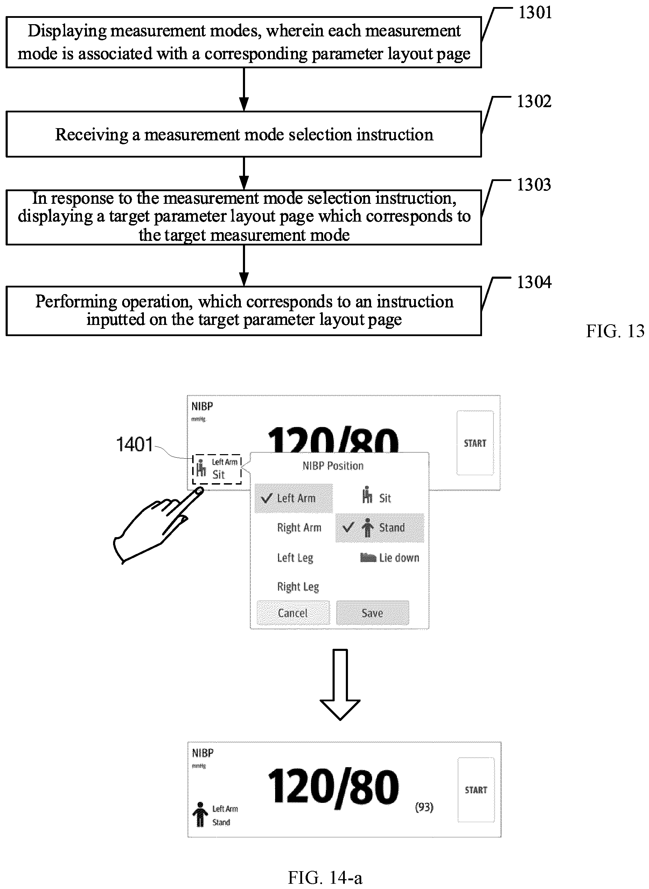

Please refer to FIG. 13. Another embodiment of the interface display method in an embodiment of this disclosure includes following steps.

In step 1301, measurement modes are displayed, wherein each measurement mode is associated with a corresponding parameter layout page.

In this embodiment, the specific way to obtain the parameter layout page can be as follows. The parameter layout page which corresponds to each measurement mode can be transmitted to the monitor, so that the monitor receives the imported parameter layout page and saves it for later retrieval and display of the target parameter layout page.

In step 1302, a measurement mode selection instruction is received.

In step 1303, a target parameter layout page which corresponds to the target measurement mode is displayed, in respond to the measurement mode selection instruction.

The operations performed in steps 1302 to 1303 are similar to those performed in steps 302 to 303 of the embodiments shown in FIG. 3, and are not repeated here.

In step 1304, operation, which corresponds to an instruction inputted on the target parameter layout page, is performed.

After displaying the target parameter layout page to the user, the user can input some instructions on the target parameter layout page, the instructions can instruct the monitor to switch the parameter measurement mode, and re-measure a certain parameter, or perform other operations. The following describes the operations performed by the monitor according to the instructions inputted by the user on the target parameter layout page.

In a specific method, the user can switch the measurement mode of a certain measurement parameter. Specifically, the monitor receives a measurement mode switching instruction inputted from the user for any measurement parameter. The measurement mode switching instruction corresponds to a target measurement mode. That is, the measurement mode switching instruction indicates that the user needs to switch the measurement mode of the measurement parameter to the target measurement mode. Wherein, this measurement parameter can be any measurement parameter in the target parameter layout page, which has multiple measurement modes.

After receiving the measurement mode switching instruction, the monitor responds to the measurement mode switching instruction, so as to switch from the current measurement mode of the measurement parameter to the target measurement mode corresponding to the measurement mode switching instruction.

For example, for NIBP parameter, according to the measurement portion of the subject, the NIBP value can be measured through the left/right arm or left/right leg. According to the posture of the subject, the NIBP values of the subject can be measured in various postures, such as standing, sitting, and lying down. There are various ways to measure the NIBP parameters.

More specifically, the monitor can receive a measurement mode switching instruction from the user in multiple manners. In one manner, the monitor displays a measurement mode icon for the measurement parameter on the target parameter layout page. The user can click on the measurement mode icon, and the monitor receives the click operation of the user and responds to it, so as to show the user at least two measurement modes for the measurement parameter. The user can select the target measurement mode from multiple measurement modes. The operation of selecting a target measurement mode among multiple measurement modes can be recognized as that the user inputs a measurement mode switching instruction, and the target measurement mode selected by the user can be determined as the corresponding target measurement mode of the measurement mode switching instruction. The monitor responds to the selection operation for the measurement mode and determines the selected measurement mode as the target measurement mode.

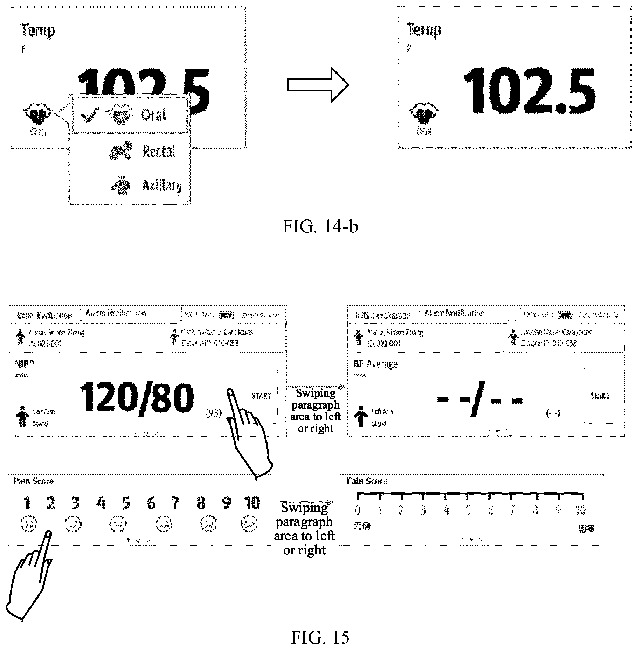

For example, FIGS. 14-a and 14-b are schematic diagrams of switching processes of measurement modes by a monitor, according to a measurement mode switching instruction. As shown in FIG. 14-a, in the NIBP display area of the target parameter layout page, icon 1401 represents the measurement mode icon for NIBP parameter. When the user clicks on this icon, the monitor responds to the click operation of the user on the measurement mode icon, so as to display multiple measurement modes for NIBP parameter. The user can select the target measurement mode from these multiple measurement modes. Assuming the user selects "Left Arm" and "Stand", that is, the subject is standing to measure left arm blood pressure. After the user clicks on the "Save" button to save, the monitor recognizes the selection operation of the user as the measurement mode switching instruction inputted by the user, and determines that the target measurement mode selected by the user is "Left Arm" and "Stand". Afterwards, the monitor responds to the user inputted measurement mode switching instruction, so as to switch from the measurement mode of NIBP parameter to "Left Arm" and "Stand".

Similarly, as shown in FIG. 14-b, in the Temp display area of the target parameter layout page, a measurement mode icon for the Temp parameter is displayed. The user can click on this icon to select the target measurement mode from multiple measurement modes. Assuming the user selects "Oral", the monitor responds to the user inputted measurement mode switching instruction, so as to switch the measurement mode of the Temp parameter to "Oral", which means measuring oral temperature.

Preferably, the measurement mode icon can be represented graphically, making the graphical representation more intuitive and clearer. The measurement mode icon can also be uniformly set in the same position in the display area, which can help users to develop usage habits and reduce learning costs, such as being uniformly set in the bottom left corner of the display area.

The measurement mode icon can also include a measurement tool identifier for selecting a measurement tool for the measurement parameter, such as CCHD measurement tool under blood oxygen, orthostatic hypotension evaluation tool under NIBP blood pressure, multiple blood pressure measurement and recording tool, and average blood pressure measurement tool. Measurement tools can be displayed through a separate display window or directly in the corresponding measurement parameter display area.

In this embodiment, the instruction inputted to the monitor can also instruct the monitor to change a display mode of the measurement parameter. Therefore, the monitor receives a display mode change instruction inputted for the measurement parameter, which is configured to change from the current display mode of the measurement parameter to a target display mode. The monitor responds to the display mode change instruction, so as to change from the current display mode of the measurement parameters to the target display mode which is indicated by the display mode change instruction.

There are various specific embodiments for the monitor to receive the display mode change instruction for the measurement parameter. In some embodiments, any one measurement parameter in the target parameter layout page has a corresponding display area, which can include any information related to the measurement parameter, such as measurement result, measurement mode, or name of the measurement parameter. Wherein, the display area of the measurement parameter is associated with area subpages of at least two display modes. The content of the area subpage includes an parameter item that need to be displayed for the measurement parameter in the corresponding display mode on the area subpage. This parameter item can include any information related to the measurement parameter, such as measurement data of the measurement parameter. The user can swipe in the display area corresponding to the measurement parameter to switch the currently displayed area subpage of the display area. As each area subpage corresponds to one display mode, the monitor recognizes the swiping operation for switching the area subpage as the display mode change instruction, and further switches the area subpage according to this display mode change instruction.

In some embodiments, the display mode can be determined by the measurement mode. As each area subpage corresponds to one display mode, when the use swipes in the display area to switch the area subpage, it is equivalent to switch the measurement mode. The swiping operation to switch the area subpage, is also recognized as the user inputted measurement mode switching instruction. In some embodiments, the display mode may also be independent of the measurement mode, that is, one measurement mode corresponds to multiple display modes, and the measurement parameter can have multiple display modes under the same measurement mode. This embodiment does not limit this.

Among them, the swiping operation of the user in the display area can be in various directions such as up and down, left and right and the swiping direction is not limited.

After receiving the display mode change instruction, the monitor responds to the instruction, so as to switch from the currently displayed area subpage of the display area of the measurement parameter to the corresponding area subpage of the target display mode.

For example, FIG. 15 is a schematic diagram of switching processes of measurement modes by a monitor, according to a measurement mode switching instruction which is inputted by swiping operation of user. As shown in the figure, there are multiple measurement modes for the blood pressure parameter, including ordinary single blood pressure measurement mode and average blood pressure measurement mode. Each measurement mode corresponds to one area subpage of the display area. The user can swipe left and right in the display area to switch the area subpage. In FIG. 15, the user swipes the display area, and the monitor switches from the currently displayed area subpage which corresponds to the ordinary single blood pressure measurement to the area subpage which corresponds to the average blood pressure measurement mode.

In some embodiments, the display mode is determined by an appearance design mode. For example, the display area of the Pain Score parameter at the bottom of FIG. 15 is also associated with area subpages of various display modes. In practical applications, doctor selects a suitable pain scoring appearance design mode according to situations of different patients. There are multiple ways to display pain scores. Therefore, when switching the appearance design mode, the user swipes the display area, and the monitor switches the currently displayed area subpage of Pain Score parameter to the corresponding area subpage of another display mode.

The above describes the situation where the user switches the measurement modes and display modes of the measurement parameter. In other specific methods, the monitor performs operations according to the instructions inputted by the user on the target parameter layout page, as follows. The monitor re-measures the measurement parameter according to the instruction of the user, and the re-measurement of the parameter can update the measurement result of the parameter. Specifically, the monitor receives a re-measurement instruction from the user, which instruction corresponds to the parameter to be updated in the target parameter layout page. Afterwards, the monitor responds to the re-measurement instruction, so as to obtain new measurement data for the parameter to be updated, and replaces the original measurement data for the parameter to be updated with the new measurement data. In some embodiments, in response to the re-measurement instruction, the current measurement data is replaced with new measurement data.

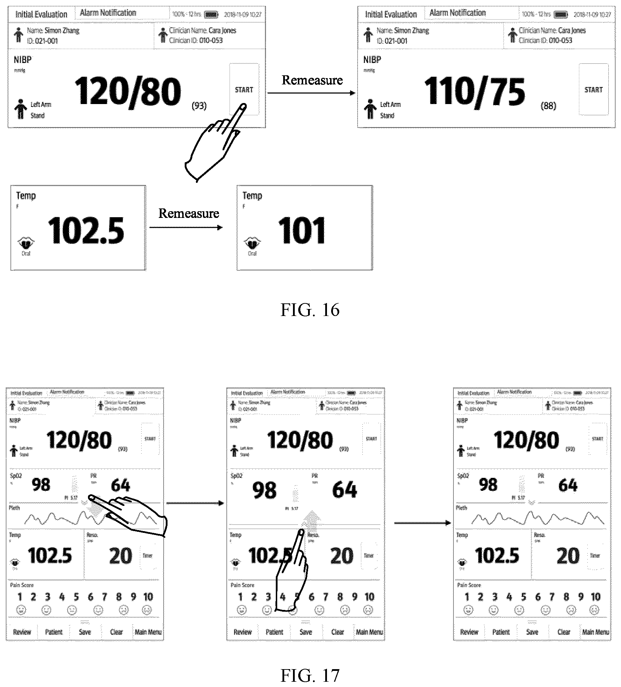

For example, FIG. 16 is a schematic diagram of a process in which a monitor re-measures updated parameters according to a re-measurement instruction. Blood pressure and body temperature parameters can be measured multiple times. When the measurement results are inaccurate due to various influencing factors during the measurement process, doctor can re-measure the blood pressure and body temperature parameters. As shown in the figure, when it is necessary to re-measure the blood pressure parameter, the user clicks the "START" button to input the re-measurement instruction. The monitor responds to the instruction, so as to re-measure the blood pressure parameter to obtain new measurement data for the blood pressure parameter. Then, the original measurement data for the blood pressure parameter is replaced by the new measurement data.

Similarly, when the user inputs a re-measurement instruction for body temperature parameter, the monitor responds to the instruction, so as to replace the original measurement data of the body temperature parameter by the obtained new measurement data.

In this embodiment, the user can re-measure the measurement parameter at any time according to the requirement of the actual situation. In one implementation, the user can re-measure the parameter under the indication of the monitor. Specifically, in this embodiment, the target parameter layout page corresponds to the target measurement mode, which includes a continuous measurement working mode. The continuous measurement working mode refers to the continuous updating of the measurement results of the parameters. For example, for some patients, they need to be continuously monitored. During the continuous monitoring process, the physiological parameters of the patient, such as blood pressure, blood oxygen, pulse rate, respiration, and body temperature, need to be continuously monitored, so as to observe changes in the physiological state of the patient. Therefore, for the continuous measurement mode, the measurement results of parameters have certain timeliness. In this embodiment, the target parameter layout page includes a manual parameter which manual inputted measurement data. The monitor can set the effective duration of the measurement data for the manual parameter according to the instruction of the user. If the time interval between the current time point and the generation time point of the measurement data for the manual parameter exceeds this effective duration, the user is indicated that the measurement data for manual parameter is invalid, and the user re-measures the manual parameter according to the indication. The monitor uses the re-measured result to update the measurement data of the manual parameter.

In other specific methods, the monitor performs operations according to the instructions inputted by the user on the target parameter layout page, as follows. the monitor opens or hides the display area according to the instruction of the user. Specifically, the measurement parameters all have corresponding display areas on the target parameter layout page. The display areas on the target parameter layout page include a first display area and a second display area. The monitor displays the first display area and receives an inputted operation instruction for the display area. When the operation instruction for the display area is to open the second display area on the target parameter layout page, the monitor responds to the operation instruction for the display area, so as to open the second display area. When the operation instruction for the display area is to hide the second display area, the monitor responds to the operation instruction for the display area, so as to hide the second display area.

Wherein, the user can input operation instructions for the display area through swiping gesture. In other ways, the monitor can also provide a "hide" or "open" button. When the user clicks on the "hide" or "open" button, the monitor perceives that the user inputs the operation instructions for the display area. This embodiment does not limit the way in which users input operation instructions for the display area.

For example, FIG. 17 is a schematic diagram of a process in which a monitor opens/hides a display area according to a display area operation instruction. As shown in the figure, the display area of the Pleth parameter displays blood oxygen waveform. In practical applications, the use of blood oxygen waveform depends on the rules and regulations of the hospital and the specific situation of the patient. For example, if some patients have irregular pulse symptoms, experienced nurses can observe the blood oxygen waveform to pay attention to whether the patient has irregular pulse conditions. For healthcare professionals who do not need to view blood oxygen waveforms, the blood oxygen waveforms in the interface may cause visual information redundancy, making it difficult for healthcare professionals to focus on more important information. Therefore, in this embodiment, the monitor provides the function for operating the display area, and the user can hide or open the display area according to actual requirements. As shown in the figure, when the user swipes down according to the arrow indication in the display area, the monitor recognizes the user inputs the operation instruction for the display area, and the instruction indicates to hide The display area of Pleth parameter. Then, the monitor responds to the instruction, so as to hide the display area of Pleth parameter. When the user swipes up according to the arrow indication, the monitor recognizes the user inputs the operation instruction for the display area, and the instruction indicates to open the display area. Then, the monitor responds to the instruction, so as to open the display area of Pleth parameter.

In this embodiment, the user can swipe from various directions such as up, down, left, and right, so as to input the operation instructions for the display area and the instruction inputted by swiping in each direction can be set independently to indicate whether to open or hide the display area. This embodiment is not limited to this.

In this embodiment, the measurement mode, which is determined according to the measurement situation and the continuous measurement working mode, corresponds to a first parameter layout page. When the target parameter layout page is the first parameter layout page, alarm limit information of the measurement parameter in the first parameter layout page is displayed. The alarm limit information is an alarm threshold range, indicating that an alarm is issued when the measurement data of the measurement parameter exceeds the alarm threshold range. For example, the alarm limit information of the SpO2 parameter is between a range of 90% and 100%. When the actual value of the SpO2 parameter measured from the subject is within the range indicated by the alarm limit information, it indicates that the physiological parameter of the subject is normal. If the actual value exceeds the range indicated by the alarm limit information, it indicates that the SpO2 parameter is abnormal and an alarm needs to be issued to indicate the user.

The alarm limit information of the measurement parameter can be displayed in any measurement mode, especially suitable for measurement mode determined according to the measurement situation and the continuous measurement working mode. This is because in the continuous measurement working mode, the monitor continuously monitors and measures the physiological parameters of the subject in real-time. However, the user may not always pay attention to the changes in the parameters. Once the physiological parameters are abnormal, it is necessary to issue an alarm for indicating to the user to pay attention to the abnormal parameters and take timely response measures.

In other specific manners, the monitor performs operations according to the instructions inputted by the user on the target parameter layout page, as follows. The user can input a locking instruction for the target parameter in the currently displayed parameter report page, which is configured to lock the layout position of the target parameter, so that the layout position of the target parameter remains unchanged.

There is no limitation to the manners for the user to input the locking instruction. For example, if the user clicks on a button which represents locking the layout position of the parameter, the monitor recognizes the click operation of the user on the button as the user inputted locking instruction. Optionally, the user presses the display area of the target parameter for a long time, and then the monitor recognizes the long press operation of the user as the user inputted locking instruction, and so on.

After receiving the locking instruction, the monitor responds to the locking instruction, so as to lock the layout position of the target parameter, such that when the parameter layout page includes at least two parameter report pages and switches between them, the layout position of the target parameter can still remain unchanged in the parameter report area.

The user can lock the layout position of the target parameter or unlock the locked layout position of the target parameter. That is, after the layout position of the target parameter is locked, the user can input an unlocking instruction for the target parameter, so that the monitor responds to the unlocking instruction, so as to unlock the locked layout position of the target parameter. Afterwards, when switching the currently displayed parameter report page, the target parameter is also switched with the switching of the parameter report page.

By locking and unlocking the layout position of the target parameter, the parameter that, the user needs to continuously pay attention to, is always displayed when switching pages, satisfying the requirement of the user for the display of the working interface of the monitor.

When the monitor is not in use, that is, it is in standby mode. At this time, the monitor can display remaining battery life and/or remaining time of the smart battery on the interface, or display pre-use precautions for the monitor.

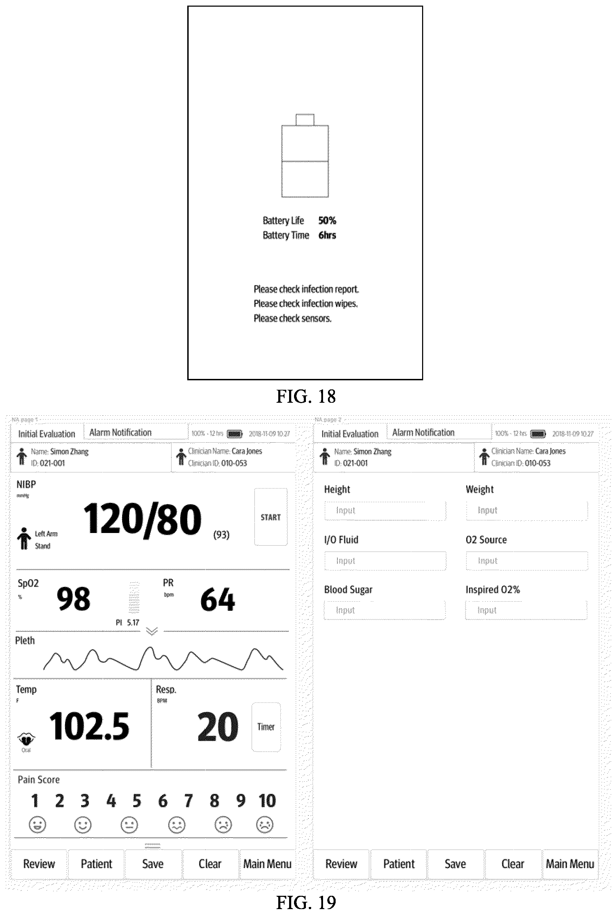

For example, FIG. 18 is an interface display diagram of a monitor in a standby mode. As shown in the figure, the monitor displays the remaining battery power, remaining usage time, and pre-use precautions the monitor in the standby mode. In practical application, when medical staff need to use a monitor, they come to the unified storage area of the device and select a device with a longer remaining time and longer battery life according to the different remaining time displayed on different device screens. At the same time, the interface of the monitor also reminds medical staff of precautions before using the monitor. Its content can include multiple aspects, such as safety and cleaning matters (hospital hygiene and disinfection requirements, probe accessory cleaning inspection, overall machine cleaning and disinfection record inspection, etc.), device availability (remaining battery life of the device, whether the device probe is complete, and whether the items in the cart basket are complete, etc.), and other precautions.

In this embodiment, after displaying the target parameter layout page, the user can directly perform some operations on the target parameter layout page, such as modifying the measurement mode of parameter, re-measuring, hiding/opening the display area, etc., which facilitates the control of the user in the parameter display and parameter measurement, and improves the user experience of using the monitor.

The above describes the display, control, editing, operation, and other processes of the parameter layout page in the embodiment of this disclosure. The following describes the parameter layout pages corresponding to several main measurement modes.

FIG. 19 is a schematic diagram of a parameter layout page which corresponds to one measurement mode. As as shown in the figure, this measurement mode is a first default measurement mode, and the parameters in the parameter layout page, which corresponds to the first default measurement mode, include one or more parameters of: blood pressure value (i.e., NIBP parameter), blood oxygen (i.e., oxygen saturation Sp02 and blood oxygen waveform Pleth), pulse rate (i.e., PR parameter), body temperature (i.e., Temp parameter), respiration (i.e., respiratory rate Resp), and Pain Score, wherein the blood pressure value includes average blood pressure value and/or ordinary single blood pressure value.

In addition, the parameter layout page which corresponds to the first default measurement mode can also include parameters, such as weight, height, blood sugar, etc.

The first default measurement mode can be applied to General Ward or Long Term Care Nursing Homes in the United States. General wards in the United States usually measure blood pressure, blood oxygen, pulse rate, body temperature of the patient, and record parameters, such as respiratory and pain scores. Therefore, the parameter layout page which corresponds to the first default measurement mode can satisfy the usage requirements of General Ward.

Wherein, the parameters in the parameter layout page, which corresponds to the first default measurement mode, include continuous monitoring parameters. For example, in certain conditions, doctor may need to constantly monitor changes of blood pressure of the patient, while the parameter of the blood pressure value needs to be continuously monitored and updated to observe the changes of blood pressure of the patient.

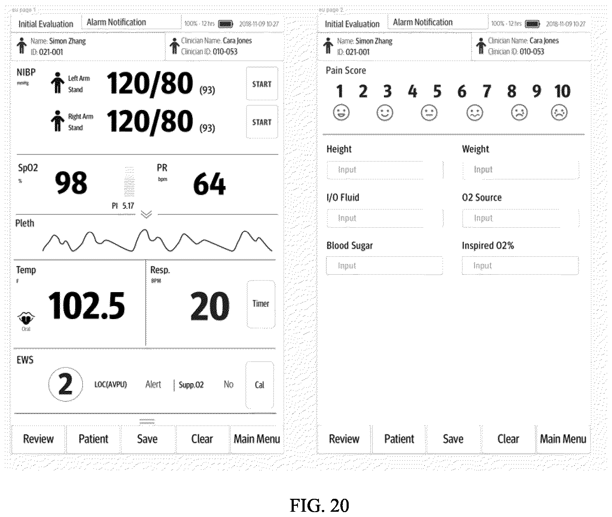

FIG. 20 is a schematic diagram of a parameter layout page which corresponds to another measurement mode. As shown in the figure, this measurement mode is a second default measurement mode, and the parameters in the parameter layout page, which corresponds to the second default measurement mode, include one or more parameters of blood pressure value, blood oxygen, pulse rate, body temperature, respiratory and pain scores, early warning score (i.e., EWS parameters), wherein the blood pressure value includes single blood pressure value and/or dual blood pressure value.

The second default measurement mode is applicable to General Wards in UK, which usually measure blood pressure, blood oxygen, pulse rate, body temperature, and record parameters, such as respiratory and pain scores. For hospitals in the UK and other European countries, dual blood pressure measurement and calculation of EWS scores are required. Therefore, for General Wards in the UK, setting dual blood pressure parameter in the display area of blood pressure value, and setting the display area of EWS scores, can satisfy the measurement requirements of General Wards.

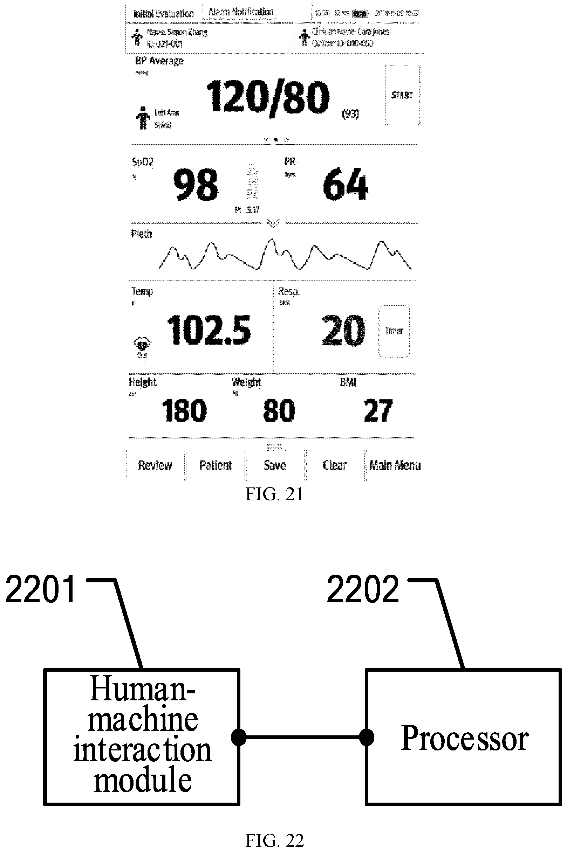

FIG. 21 is a schematic diagram of a parameter layout page which corresponds to another measurement mode. As shown in the figure, this measurement mode is a third default measurement mode, and the parameters in the parameter layout page, which corresponds to the third default measurement mode, include one or more parameters of blood pressure value, blood oxygen, pulse rate, body temperature, respiration and height, weight, body mass index (i.e., BMI parameters), wherein the blood pressure value includes average blood pressure value and/or ordinary single blood pressure value.

The third default measurement mode can be applied to Doctor Clinics, which usually measure blood pressure, blood oxygen, pulse rate, body temperature, and record parameter, such as respiratory parameter. However, unlike other departments, doctor in this department pays special attention to the height, weight, BMI index of the patient, and may measure average blood pressure when measuring blood pressure. Therefore, this department may need to be equipped with both average blood pressure measurement mode and ordinary single blood pressure measurement mode which can be switched according to the patient and the habit of the doctor.

The above introduces several main default measurement modes. In practical applications, the corresponding measurement modes can be performed by default according to the actual usage situations of the monitor. For example, when the monitor is used in a General Ward in the United States, it can be configured to display the parameter layout page which corresponds to the first default measurement mode by default when the monitor is turned on. The user does not need to select the measurement mode, which is convenient for the use of the monitor.

The interface display method in the embodiment of this disclosure has been described above, and the monitor in the embodiment of this disclosure is described below, please refer to FIG. 22. One embodiment of the monitor in the embodiment of this disclosure includes: a human-machine interaction module 2201 and a processor 2202.

The human-machine interaction module 2201 is configured to receive a measurement mode selection instruction and/or a selection instruction for a functional control element, which are/is inputted by a user, and the human-machine interaction module 2201 is further configured to display a work interface.

The processor 2202 is configured to respond to a selection instruction for the functional control element, so as to execute a processing flow which corresponds to a selected target functional control element.

The work interface includes:
a mode selection area, which is configured to display measurement modes and to receive a measurement mode selection instruction; the measurement modes are at least configured to characterize a working mode of the monitor, wherein the working mode includes a continuous measurement working mode and a discontinuous measurement working mode; configured to determine multiple first measurement modes according to the continuous measurement working mode, wherein the multiple first measurement modes respectively correspond to multiple first parameter layout pages; configured to determine multiple second measurement modes according to the discontinuous measurement working mode, wherein the multiple second measurement modes respectively correspond to multiple second parameter layout pages; wherein the first parameter layout pages are at least partially different from the second parameter layout pages; the measurement mode selection instruction is configured to select a target measurement mode from a measurement mode identifier list, wherein the measurement mode identifier list includes the multiple first measurement modes and the multiple second measurement modes;
a state display area, which is configured to display state information of the monitor;
a parameter report area, which is configured to display a target parameter layout page which corresponds to the target measurement mode, according to the target measurement mode; wherein the target parameter layout page includes at least one display area of a measurement parameter and measurement data which is located within said display area; wherein when the target parameter layout page belongs to the first parameter layout pages, the target parameter layout page includes a first parameter report area; when the target parameter layout page belongs to the second parameter layout pages, the target parameter layout page includes a second parameter report area, wherein the first parameter report area is at least partially different from the second parameter report area;
a functional control element area, which includes at least one functional control element.

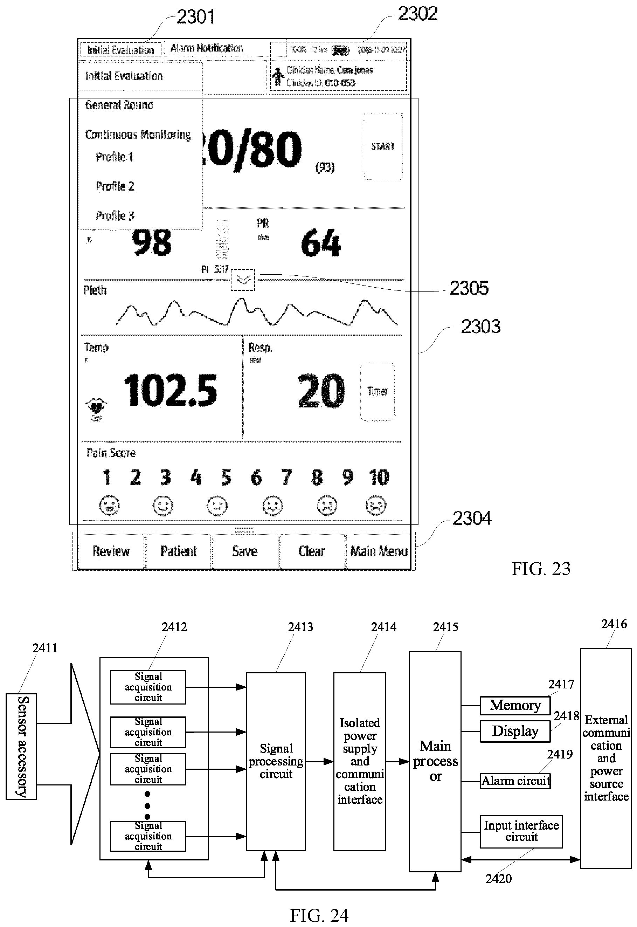

Takes the schematic diagram of the work interface shown in FIG. 23 as an example, the work interface of the monitor in this embodiment is explained in detail. As shown in the figure, a first mode selection area 2301 is associated with at least one measurement mode. When the user clicks on the first mode selection area 2301, the working interface displays multiple measurement modes for the user to select. As shown in the figure, the corresponding identifiers of multiple measurement modes, such as "General Round" and "Continuous Monitoring", are displayed.

A first state display area 2302 displays state information of the monitor, which can include any information related to the state of the monitor, such as battery life information, time information of the monitor, or user information when the monitor is in use.

A first parameter report area 2303 is configured to display the parameter layout page, which can display at least one display area of a measurement parameter and measurement data which is located within said display area, measurement mode of the measurement parameter, and other information. The parameter layout page can include at least one parameter report page, and each parameter report page includes at least one display area. When the parameter layout page includes at least two parameter report pages, the first parameter report area 2303 displays one of the parameter report pages. Switching between the at least two parameter report pages can be carried out according to a parameter report page selection instruction, as mentioned earlier. The parameter displayed in the display area can include physiological parameter, evaluation result, and other related information, and the parameter displayed is not limited here. In some embodiments, FIG. 23 shows that there are six display areas distributed on the parameter report page of the parameter layout page, including NIBP blood pressure display area, SPO2 blood oxygen display area, PR pulse rate display area, Temp temperature display area, Resp respiratory rate display area and pain score display area.

A first functional control element area 2304 includes at least one functional control element, such as, Review control element (review functional control element), Patient control element (case reference functional control element), Save control element (save functional control element), and Clear control element (clearing functional control element) shown in FIG. 23.

It should be noted that specific information in the work interface shown in FIG. 23 (such as specific parameters, specific functional control elements, and specific state information) is only for the convenience of illustration and does not necessarily represent the content that the work interface of the monitor in this embodiment displays. The work interface shown in FIG. 23 does not constitute a limitation of this embodiment.

The user can input a selection instruction for the functional control element through human-machine interaction module 2201 to select a target functional control element, and then processor 2202 executes the corresponding processing flow of the target functional control element. Specifically, when the user clicks on the review functional control element, the processor displays a historical record of the parameter measurement data stored by the monitor to the user. When the user clicks on the case reference functional control element, the processor displays the parameter measurement data of the target patient to the user. When the user clicks on the save functional control element, the processor saves the measurement data on the target parameter layout page. When the user clicks on the clearing functional control element, the processor clears the measurement data on the target parameter layout page.

The display area of the measurement parameters includes a measurement mode icon for displaying the measurement mode of the measurement parameter. The measurement mode icon is further configured to display at least two measurement modes of the measurement parameters after receiving click operation, for selecting one of the measurement modes as the target measurement mode.

In some embodiments, the display area of the measurement parameter includes at least two area subpages, each of which is configured to display the measurement parameter in a corresponding display mode. The at least two area subpages are switched according to the display mode change instruction to determine the target display mode.

In some embodiments, the display area of the measurement parameter also includes a re-measurement control element which is configured to receive an inputted re-measurement instruction and to replace a current measurement data with new measurement data.

In some embodiments, the display area of the measurement parameter also includes an open control element 2305 which is configured to open a hidden area of the display area of the measurement parameter when a display area operation instruction of the measurement parameter is to open the display area of the measurement parameter.

When receiving an operation instruction for the display area of the measurement parameter to hide the display area of the measurement parameter, the hidden area of the measurement parameter is hidden.

In some embodiments, when receiving an inputted operation instruction for the display area, the display area of the measurement parameter is specifically configured to receive the operation instruction for the display area of the measurement parameter which is inputted through swiping gesture.

In some embodiments, when the parameter layout page includes at least two parameter report pages, the parameter report area displays one parameter report page, and the at least two parameter report pages are switched in the parameter report area.

In some embodiments, the display area of the measurement parameter also includes a locking control element for receiving a locking instruction for a target parameter in the currently displayed parameter layout page. The locking instruction is configured to lock a layout position of the target parameter, such that when including at least two parameter report pages by the parameter layout page, and switching between the at least two parameter report pages, the layout position of the target parameter remains unchanged in the parameter report area.

In some embodiments, the locking control element is further configured to unlock the layout position of the target parameter according to a received unlocking instruction; wherein when switching between at least two parameter report pages, the layout position of the target parameter changes in the parameter report area with the switching of the at least two parameter report pages.

In some embodiments, the first parameter layout page includes a first functional control element area, the second parameter layout page includes a second functional control element area, and a first functional control element in the first functional control element area is at least partially different from a second functional control element in the second functional control element area.

In some embodiments, the first functional control element area includes at least one of a review functional control element, a case reference functional control element, a save functional control element, and a clearing functional control element; wherein:
the review functional control element is configured to display a historical record of parameter measurement data stored by the monitor,
the case reference functional control element is configured to display parameter measurement data of a target patient;
the save functional control element is configured to save the measurement data on the target parameter layout page;
the clearing functional control element is configured to clear the measurement data on the target parameter layout page.

In some embodiments, the mode selection area is specifically configured to display a measurement mode identifier list according to a measurement situation of the monitor, wherein the measurement mode identifier list includes an identifier for each measurement mode in the measurement situation;
the human-machine interaction module is specifically configured to receive a target identifier selected through the measurement mode identifier list, wherein the target identifier corresponds to the target measurement mode;
wherein, the measurement situation includes a hospital working subarea, which at least includes: General Ward and Emergency Department;
when the measurement situation is general ward, a continuous measurement working mode in the measurement mode identifier list includes a continuous monitoring mode, and a discontinuous measurement working mode in the measurement mode identifier list includes a general round mode and an initial evaluation mode;
when the measurement situation is Emergency Department, a continuous measurement working mode in the measurement mode identifier list includes a continuous monitoring mode, and a discontinuous measurement working mode in the measurement mode identifier list includes a triage mode and a spot check mode.

In another preferred embodiment, the parameter report area also includes a parameter measurement indication identifier, which is configured to provide an measurement indication for at least one of at least two measurement parameters according to a measurement order of the at least two measurement parameters.

In another preferred embodiment, the parameter measurement indication identifier is further configured to:
distinguishingly display a display area of a target measurement parameter and display area(s) which is (are) in the target parameter layout page but other than the display area of the measurement parameter which has the measurement priority;
wherein, the target measurement parameter is a measurement parameter that need to be measured currently, and that is determined from unmeasured measurement parameters, according to the measurement order and ranking positions of currently measured measurement parameters in the measurement order.

In another preferred embodiment, the parameter measurement indication identifier includes at least one of: a border of the display area of the measurement parameter, and a color of the display area of the measurement parameter.

In another preferred embodiment, the parameter measurement indication identifier is further configured to:
mark a display area which is unfilled with measurement data on the target parameter layout page as unfilled; and/or,
when receiving measurement data of the target measurement parameter in the at least one measurement parameter, and filling the measurement data of the target measurement parameter into a corresponding display area of the target measurement parameter, mark the display area of the target measurement parameter as filled.

In another preferred embodiment, the functional control element area further includes an editing functional control element;
in order to respond to a selection instruction for the functional control element, so as to execute a processing flow which corresponds to a selected target functional control element, the processor is specifically configured to:
when receiving click operation of the user on the editing functional control element through the human-machine interaction module, display an editing page to the user through the work interface, receive editing information for the editing page, which editing information is configured to represent relevant operation information for the measurement parameter on the editing page, and operate the measurement parameter on the editing page according to the editing information, so as to obtain a parameter layout page,.

In another preferred embodiment, the editing information includes parameter layout information, which is configured to represent layout information of several measurement parameters on the editing page;
wherein in order to operate the measurement parameter on the editing page according to the editing information, so as to obtain a parameter layout page, the processor is specifically configured to:
do layout of several measurement parameters on the editing page according to the parameter layout information, so as to obtain the parameter layout page.

In another preferred embodiment, the editing information includes change information of measurement parameter, which change information is configured to change the measurement parameter in the editing page to a target parameter;
wherein in order to operate the measurement parameter on the editing page according to the editing information, so as to obtain a parameter layout page, the processor is specifically configured to:
change the measurement parameter to the target parameter according to the change information, so as to obtain the parameter layout page.

In another preferred embodiment, wherein in order to receive editing information for the editing page, the processor is specifically configured to:
display an optional parameter list to the user, wherein the optional parameter list includes multiple optional parameters;
receive a target parameter which is selected by the user through the optional parameter list, wherein the target parameter is one of the multiple optional parameters.

In another preferred embodiment, the editing information includes position change information of measurement parameter on the editing page, which position change information is configured to represent a change of layout position of the measurement parameter on the editing page;
wherein in order to operate the measurement parameter on the editing page according to the editing information, so as to obtain a parameter layout page, the processor is specifically configured to:
change the layout position of the measurement parameter on the editing page according to the position change information, so as to obtain the parameter layout page.

In another preferred embodiment, wherein in order to receive editing information for the editing page, the processor is specifically configured to:
receive gesture dragging operation inputted by a user, which gesture dragging operation is configured to move the measurement parameter to a target position;
in order to change the layout position of the measurement parameter on the editing page according to the position change information, so as to obtain the parameter layout page, the processor is specifically configured to:
   move the measurement parameter from a current layout position to a target position according to the gesture dragging operation.

In another preferred embodiment, the editing information includes measurement setting information of the measurement parameter, and the measurement setting information is configured to set a measurement mode of the measurement parameter;
wherein in order to operate the measurement parameter on the editing page according to the editing information, so as to obtain a parameter layout page, the processor is specifically configured to:
set the measurement mode of the measurement parameter to the measurement mode, which corresponds to the measurement setting information, according to the measurement setting information, so as to obtain the parameter layout page.

In another preferred embodiment, the editing information includes addition information of the measurement parameter, which is configured to add a measurement parameter on the editing page;
wherein in order to operate the measurement parameter on the editing page according to the editing information, so as to obtain a parameter layout page, the processor is specifically configured to:
add the measurement parameter to the editing page according to the addition information, so as to obtain the parameter layout page.

In another preferred embodiment, the measurement parameter include a parameter with manually inputted measurement data, and the editing information includes a type setting information of the measurement parameter, which type setting information is configured to set a parameter type of the measurement parameter, wherein the parameter type includes a numeric type and an optional type;
wherein in order to operate the measurement parameter on the editing page according to the editing information, so as to obtain a parameter layout page, the processor is specifically configured to:
set the parameter type of the measurement parameter to a parameter type which corresponds to the type setting information, so as to obtain the parameter layout page.

In another preferred embodiment, in order to set the parameter type of the measurement parameter to the parameter type which corresponds to the type setting information, so as to obtain the parameter layout page, the processor is specifically configured to:
set the parameter type of the measurement parameter to the numeric type;
wherein the processor is further configured to:
   receive detailed setting information of the measurement parameter, which detailed setting information is configured to set parameter detail of the measurement parameter, wherein the parameter detail includes one or more items of numeric unit, numeric input range, and numeric accuracy; and
   set the parameter detail of the measurement parameter to corresponding parameter detail, according to the detailed setting information.

In another preferred embodiment, in order to set the parameter type of the measurement parameter to the parameter type which corresponds to the type setting information, so as to obtain the parameter layout page, the processor is specifically configured to:
set the parameter type of the measurement parameter to the optional type;
wherein the processor is further configured to:
   set multiple options related to the measurement data of measurement parameter.

In another preferred embodiment, the processor is further configured to:
display to the user through the work interface, an effect preview page of the edited editing page.

In some embodiments, the display area of the measurement parameter is further configured to receive at least one of a measurement mode switching instruction and a display mode change instruction which are inputted for the measurement parameter; in response to the measurement mode switching instruction, so as to switch from a current measurement mode of the measurement parameter displayed in the display area of the measurement parameter to a target measurement mode of the measurement parameter; and in response to the display mode change instruction, so as to switch from a current display mode of the measurement parameter displayed in the display area of the measurement parameter to a target display mode of the measurement parameter.

In this embodiment, the operations performed by the human-machine interaction module 2201 and processor 2202 are similar to those described in the aforementioned embodiments shown in FIGS. 2, 3, and 13, and are not repeated here.

In another preferred embodiment, the target measurement mode is the first default measurement mode, and the parameter report area is specifically configured to display the parameter layout page which corresponds to the first default measurement mode.

The parameters in the parameter layout page, which corresponds to the first default measurement mode, include one or more parameters of: blood pressure value, blood oxygen, pulse rate, body temperature, respiration and Pain Score, wherein the blood pressure value includes average blood pressure value and/or ordinary single blood pressure value.

In another preferred embodiment, the target measurement mode is the second default measurement mode, and the parameter report area is specifically configured to display the parameter layout page which corresponds to the second default measurement mode.

The parameters in the parameter layout page, which corresponds to the second default measurement mode, include one or more parameters of: blood pressure value, blood oxygen, pulse rate, body temperature, respiratory and pain score, early warning score, wherein the blood pressure value includes single blood pressure value and/or dual blood pressure value.

In another preferred embodiment, the target measurement mode is the third default measurement mode, and the parameter report area is specifically configured to display the parameter layout page which corresponds to the third default measurement mode;

The parameters in the parameter layout page, which corresponds to the third default measurement mode, include one or more parameters of: blood pressure value, blood oxygen, pulse rate, body temperature, respiration and height, weight, and body mass index. The blood pressure value includes average blood pressure value and/or ordinary single blood pressure value.

Wherein, the parameter layout pages corresponding to the first default measurement mode, the second default measurement mode, and the third default measurement mode have been described in the aforementioned embodiments, and are not repeated here.

In this embodiment, users can select the target measurement mode according to the usage situation of the monitor or their own work habits. After the human-machine interaction module 2201 receives the measurement mode selection instruction input by the user, the processor 2202 determines the target parameter layout page that the user needs to display according to the measurement mode selection instruction, and the parameter report area displays the target parameter layout page to the user. Therefore, the monitor can display different parameter layout pages according to different usage situations or user work habits, satisfying the different measurement requirements of users, making the monitor adaptable to their usage situations.

According to the embodiments shown in FIGS. 22 and 23, this disclosure also provides another embodiment of an interface display method. Another embodiment of the interface display method in an embodiment of this disclosure includes following steps:
displaying a work interface by a monitor, wherein the work interface includes at least one of the following: a mode selection area, a state display area, a parameter report area, and a functional control element area;
displaying at least one measurement mode according to the mode selection area, so as to obtain a parameter layout page which corresponds to each measurement mode; determining the measurement mode according to a measurement situation and a working mode, wherein the working mode includes a continuous measurement working mode and a discontinuous measurement working mode; determining a first parameter layout page according to the measurement situation and the working mode, which is determined by the continuous measurement working mode; determining a second parameter layout page according to the measurement situation and the working mode, which is determined by the discontinuous measurement working mode; wherein the first parameter layout page is at least partially different from the second parameter layout page;
displaying at least state information of the monitor according to the state display area;
displaying a target parameter layout page which corresponds to a target measurement mode, based on a parameter report area, in respond to a selection instruction for the target measurement mode from at least one measurement mode; wherein the target parameter layout page includes at least one display area of a measurement parameter and measurement data located within the display area; and
displaying at least one functional control element based on the functional control element area;
executing a processing flow which corresponds to a selected target functional control element, in respond to a selection instruction for a functional control element from at least one functional control element.

The working interface in this embodiment is similar to the one described in the previous embodiments shown in FIGS. 22 and 23, and are not repeated here.

A monitor in an embodiment of this disclosure is described as follows. Please refer to FIG. 24, which relates to the system framework diagram of a parameter processing module in a monitor. The parameter processing module is a module configured to process parameters in the monitor. One embodiment of the monitor in an embodiment of this disclosure is as follows.

The monitor has an independent housing, with a sensor interface area on the housing panel, which integrates multiple sensor interfaces for connecting to various external physiological parameter sensor accessories 2411. The housing panel also includes a small IXD display area, a display 2418, an input interface circuit 2420, and an alarm circuit 2419 (such as LED alarm zone).

Wherein, the parameter processing module of the monitor is used for external communication with and power supply acquisition from a host. The parameter processing module also supports a plug-in parameter module, or can form a plug-in monitor by inserting into the host of the monitor and serving as a part of the monitor, or can be connected with the host of the monitor via a cable and taking the build-out parameter module as an external accessory of the monitor.

The internal circuit of the parameter processing module is placed inside the housing, as shown in FIG. 24, including signal acquisition circuits 2412 corresponding to at least two physiological parameters, a front-end signal processing circuit 2413, and a main processor 2415. The signal acquisition circuits 2412 can be selected from an electrocardiogram circuit, respiratory circuit, body temperature circuit, blood oxygen circuit, non-invasive blood pressure circuit, invasive blood pressure circuit, etc. These signal acquisition circuits 2412 are respectively electrically connected to the corresponding sensor interfaces, for electrical connection to sensor accessories 2411 which correspond to different physiological parameters. Output terminals of the signal acquisition circuits 2412 are coupled to a front-end signal processor, a communication port of the front-end signal processor is coupled to the main processor, and the main processor is electrically connected to an external communication and power supply interface. Various physiological parameter measurement circuits can adopt universal circuits in existing technologies. The front-end signal processor completes sampling and analog-to-digital conversion for signals which are outputted from the signal acquisition circuits, and outputs control signals to control measurement processes of physiological signals. These parameters include but are not limited to: electrocardiogram parameter, respiratory parameter, body temperature parameter, blood oxygen parameter, non-invasive blood pressure parameter, and invasive blood pressure parameter. The front-end signal processor can be implemented using microcontrollers or other semiconductor devices, such as composite signal single chip microcomputer (such as LPC2136 from PHLIPS or ADuC7021 from ADI), or ASIC or FPGA. The front-end signal processor can be powered by an isolated power supply. After simple processing and packaging, the sampled data is transmitted to the main processor through an isolated communication interface. For example, the front-end signal processor circuit can be coupled to the main processor 2415 through an isolated power supply and communication interface 2414. Supplying electrical power to the front-end signal processing circuit through the isolated power supply has a function of isolating the patient from the power supply device through isolating the DC/DC power supply via a transformer. In such a way, the application part is floating through the isolation transformer, such that the leakage current passing through the patient is small enough, and bad influences on boards and devices of intermediate circuits, such as main control board (guaranteed by creepage distance and electrical clearance), due to voltage or energy generated during a defibrillation or electric knife application, can be prevented. The main processor completes calculation of physiological parameters, and transmits calculation results and waveforms of parameters to the host through the external communication and power supply interface (such as the host with display, PC, central station, etc.). The external communication and power source interface 2416 may be one or a combination of local area network interfaces composed of Ethernet, Token Ring, Token Bus, and optical fiber distributed data interface (FDDI) as the backbone of these three networks, may also be one or a combination of wireless interfaces such as infrared, Bluetooth, WIFI, and WMTS communication, or may also be one or a combination of wired data connection interfaces such as RS232 and USB. The external communication and power source interface 2416 may also be one or a combination of the wireless data transmission interface and the wired data transmission interface. The host may be any computer device such as a host of the monitor, an electrocardiograph, an ultrasonic diagnosis instrument, a computer, etc. A monitor can be formed by means of installing them with matching software. The host may also be a communication device, such as a mobile phone, and the parameter processing module sends data to a mobile phone that supports Bluetooth communication via a Bluetooth interface to realize remote data transmission.

Each of the aforementioned units or modules configured to execute each step can be stored in one or more memory 2417. In the aforementioned embodiments for implementing the aforementioned monitor or monitoring system, each functional module includes an instruction set for executing the corresponding steps in the interface display method shown in FIGS.2, 3, and 13. The above modules or programs (i.e., instruction sets) do not require to be constraint to be discrete software programs, processes, or modules. Therefore, in various embodiments, each submodule of these modules can be combined or rearranged. Therefore, in some embodiments of this disclosure, the memory 2417 can store subsets of modules or data structures as described above.

An embodiment of this disclosure also provides a computer storage medium, wherein in one embodiment, the computer storage medium stores instructions, when the instructions are performed on the computer, enabling a computer to perform operations which are performed by the monitor in the embodiments shown in FIGS. 2, 3, and 13.

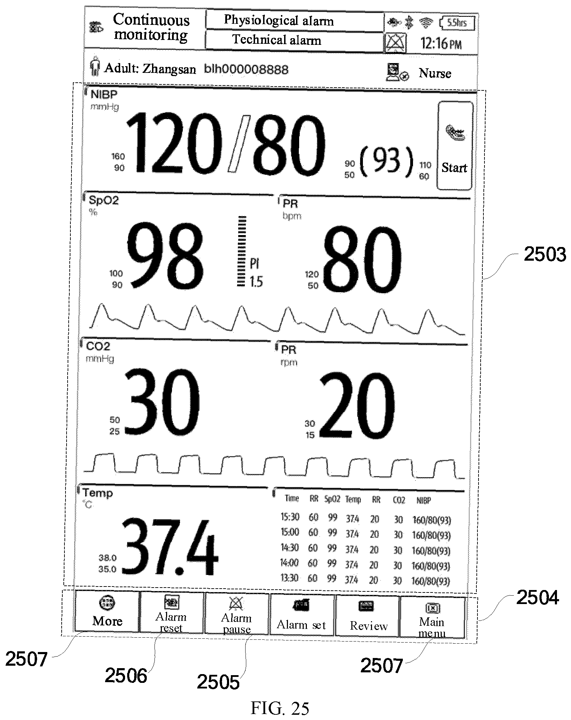

Taking a schematic diagram of awork interface shown in FIG. 25 as an example, the work interface of the monitor in an embodiment of this disclosure is further explained in detail. FIG. 25 shows an work interface in a continuous monitoring mode in an embodiment of this disclosure. As shown in FIG. 25, in some embodiments, in the continuous monitoring mode, the second parameter report area 2503 is different from the first parameter report area 2303 in spot check working mode in FIG. 23. In some embodiments, the second parameter report area 2503 is further configured to display alarm limit information. In some embodiments, the second parameter report area 2503 is further configured to display parameter trend data, and the display area is configured to display parameter trend data, such as bar charts, waveforms, etc.

In some embodiments, the second functional control element included in the second functional control element area 2504 is at least partially different from the first functional control element in the aforementioned first functional control element area 2304. The second functional control element area 2504 includes an alarm functional control element and an extension functional control element 2507. The alarm functional control element further includes an alarm pause functional control element 2505 and an alarm reset functional control element 2506. Wherein, the alarm pause functional control element 2505 is configured to control the monitor to not issue an alarm within a preset time, and the alarm reset functional control element 2506 is configured to control the monitor to reset a current alarm. The extension functional control element 2507 can view and use more functional control elements, such as a main menu.

In continuous monitoring mode, the second parameter report area 2503 can include at least one display area in the first parameter report area 2303 under the spot check working mode shown in FIG. 23. For example, the first parameter report area 2303 includes NIBP blood pressure display area, and the second parameter report area 2503 also includes the NIBP blood pressure display area. In some embodiments, the size of the display area and/or the size of the front in the same display area, are same in the second parameter report area 2503 and the first parameter report area 2303. For example, the size of the NIBP blood pressure display area and the font size inside the NIBP blood pressure display area, which are included in the first parameter report area 2303, are the same as those of the NIBP blood pressure display area included in the second parameter report area 2503. In some embodiments, the font size in the display area can be adjusted.

The parameters in this disclosure include physiological parameters, evaluation results, and other directly or indirectly measured results.

Those skilled in the art can clearly understand that for the convenience and conciseness of the description, the specific working processes of the system, device, and unit described above can refer to the corresponding processes in the aforementioned method embodiments, and are not repeated here.

In the several embodiments provided in this disclosure, it should be understood that the disclosed system, device, and method can be implemented in other ways. For example, the device embodiments described above are only schematic. For example, the division of the units is only a logical functional division, and there may be other division methods in actual implementation. For example, multiple units or components can be combined or integrated into another system, or some features can be ignored or not executed. On the other hand, the coupling or direct coupling or communication connection displayed or discussed between each other can be indirect coupling or communication connection through some interfaces, devices or units, which can be electrical, mechanical or other forms.

The units described as separate components can be or may not be physically separated, and the components displayed as units can be or may not be physical units, that is, they can be located in one place or distributed across multiple network units. Some or all of the units can be selected according to actual requirements to achieve the purpose of this embodiment.

In addition, each functional unit in each embodiment of this disclosure can be integrated in one processing unit, or each unit can exist physically independently, or two or more units can be integrated in one unit. The integrated units mentioned above can be implemented in both hardware and software functional units.

If the integrated unit is implemented in the form of a software functional unit and sold or used as an independent product, it can be stored in a computer readable storage medium. According to this understanding, the technical solution of this disclosure in essence, or the portion that contributes to the existing technology of the technical solution of this disclosure, or all or part of the technical solution of this disclosure, can be reflected in the form of a software product, which is stored in a storage medium, including several instructions to enable a computer device (which can be a personal computer, server, or network device, etc.) to perform all or some of the steps of the methods described in various embodiments of this disclosure. The aforementioned storage media include: USB flash drive, removable hard drive, read-only memory (ROM), random access memory (RAM), magnetic disk or optical disk, and other media that can store program codes.

## Claims

1. An interface display method which is applied to a monitor, **characterized in that**, comprising:
displaying a work interface on a display screen of the monitor;
displaying measurement modes in the work interface, wherein each measurement mode is associated with a corresponding parameter layout page; the measurement modes are at least configured to characterize a working mode of the monitor, wherein the working mode comprises a continuous measurement working mode and a discontinuous measurement working mode; determining multiple first measurement modes according to the continuous measurement working mode, wherein the multiple first measurement modes respectively correspond to multiple first parameter layout pages; determining multiple second measurement modes according to the discontinuous measurement working mode, wherein the multiple second measurement modes respectively correspond to multiple second parameter layout pages; wherein the first parameter layout pages are at least partially different from the second parameter layout pages;
receiving a measurement mode selection instruction, which is configured to select a target measurement mode from a measurement mode identifier list, wherein the measurement mode identifier list comprises the multiple first measurement modes and the multiple second measurement modes; and
in response to the measurement mode selection instruction, displaying a target parameter layout page which corresponds to the target measurement mode.

2. The interface display method according to claim 1, **characterized in that**, a measurement situation of the monitor comprises hospital working areas, which at least comprise General Ward and Emergency Department;
when the measurement situation is General Ward, the continuous measurement working mode comprises a continuous monitoring mode, the discontinuous measurement working mode comprises a general round mode and an initial evaluation mode;
when the measurement situation is Emergency Department, the continuous measurement working mode comprises a continuous monitoring mode, the discontinuous measurement working mode comprises a triage mode and a spot check mode.

3. The interface display method according to claim 1, **characterized in that**, receiving a measurement mode selection instruction, comprises:
displaying the measurement mode identifier list, wherein the measurement mode identifier list comprises identifier(s) of measurement mode, wherein the identifier(s) comprise(s) name(s) of measurement situation; and
receiving a target identifier, which is selected by a user through the measurement mode identifier list, wherein the target identifier corresponds to the target measurement mode.

4. The interface display method according to claim 1, **characterized in that**, after displaying a target parameter layout page which corresponds to the target measurement mode, the interface display method further comprises:
displaying a parameter measurement indication identifier to indicate measurement for at least one of at least two measurement parameters, when the target parameter layout page comprises the at least two measurement parameters.

5. The interface display method according to claim 4, **characterized in that**, displaying a parameter measurement indication identifier, comprises:
obtaining a measurement order which is preset for the at least two measurement parameters;
determining a measurement parameter which has a measurement priority, from unmeasured measurement parameter(s) in the at least two measurement parameters, according to the measurement order and a ranking position of a most recently measured measurement parameter in the measurement order, wherein the measurement parameter which has the measurement priority is a measurement parameter to be measured currently; and
displaying, on the target parameter layout page, the parameter measurement indication identifier for the measurement parameter which has the measurement priority.

6. The interface display method according to claim 5, **characterized in that**, displaying the parameter measurement indication identifier for the measurement parameter which has the measurement priority, comprises at least one of
distinguishingly displaying the measurement parameter which has the measurement priority, and measurement parameter(s), which is/(are) not the measurement parameter which has the measurement priority and is/(are) on the target parameter layout page; and
distinguishingly displaying a display area of the measurement parameter which has the measurement priority, and display area(s) of the measurement parameter(s), which is/(are) not the measurement parameter which has the measurement priority, wherein the display area(s) is/(are) on the target parameter layout page.

7. The interface display method according to claim 4, **characterized in that**, comprising:
receiving measurement data of a target measurement parameter in the at least two measurement parameters, wherein each measurement parameter of the at least two measurement parameters has a corresponding display area on the target parameter layout page;
filling the measurement data of the target measurement parameter into the display area which corresponds to the target measurement parameter; and
marking the display area of the target measurement parameter as filled, so as to distinguishingly display the display area of the target measurement parameter and a display area which is unfilled with measurement data.

8. The interface display method according to claim 7, **characterized in that**, distinguishingly displaying the display area of the target measurement parameter and a display area which is unfilled with measurement data, comprises:
displaying, in different colors, a background of the display area of the target measurement parameter and a background of the display area which is unfilled with measurement data.

9. The interface display method according to any one of claims 1-8, **characterized in that**, comprising:
receiving a measurement mode switching instruction, which is inputted for a measurement parameter, wherein the measurement mode switching instruction is configured to switch from a current measurement mode to a target measurement mode; and
in response to the measurement mode switching instruction, switching from the current measurement mode of the measurement parameter to the target measurement mode, which is indicated by the measurement mode switching instruction.

10. The interface display method according to claim 9, **characterized in that**, receiving a measurement mode switching instruction, which is inputted for a measurement parameter, comprises:
displaying, on the target parameter layout page, a measurement mode icon for the measurement parameter;
receiving click operation on the measurement mode icon for the measurement parameter;
in response to the click operation, displaying at least two measurement modes of the measurement parameter; and
in response to operation for selecting one measurement mode from the at least two measurement modes, determining the selected measurement mode as the target measurement mode.

11. The interface display method according to any one of claims 1-8, **characterized in that**, further comprising:
receiving a display mode change instruction for a measurement parameter, wherein the display mode change instruction is configured to change from a current display mode to a target display mode; and
in response to the display mode change instruction, changing from the current display mode of the measurement parameter to the target display mode, which is indicated by the display mode change instruction.

12. The interface display method according to claim 11, **characterized in that**, the measurement parameter has a corresponding display area on the target parameter layout page;
receiving a display mode change instruction for a measurement parameter, comprises:
receiving the display mode change instruction which is inputted in the display area of the measurement parameter, wherein the display area of the measurement parameter is associated with area subpages of at least two display modes of the measurement parameter, wherein each area subpage comprises measurement data of the measurement parameter, in a corresponding display mode of said area subpage;
in response to the display mode change instruction, changing from the current display mode of the measurement parameter to the target display mode, which is indicated by the display mode change instruction, comprises:
in response to the display mode change instruction, changing from a currently displayed area subpage of the display area of the measurement parameter to a corresponding area subpage of the target display mode.

13. The interface display method according to any one of claims 1-8, **characterized in that**, a measurement parameter has a corresponding display area on the target parameter layout page, wherein the display area comprises a first display area and a second display area;
wherein the interface display method further comprises:
displaying the first display area, and receiving a display area operation instruction which is inputted by a user;
in response to the display area operation instruction, opening the second display area, when the display area operation instruction is an instruction to open the second display area on the target parameter layout page; and
in response to the display area operation instruction, hiding the second display area, when the display area operation instruction is an instruction to hide the second display area on the target parameter layout page.

14. The interface display method according to claim 1, **characterized in that**, when the target parameter layout page belongs to the first parameter layout pages, the interface display method further comprises:
displaying alarm limit information of measurement parameter(s) in the first parameter layout pages.

15. The interface display method according to claim 1, **characterized in that**, further comprising:
displaying pre-use precautions for the monitor, when the monitor is in a standby mode.

16. The interface display method according to claim 1, **characterized in that**:
a parameter report area of the monitor comprises a preset number of display line(s), wherein the interface display method further comprises:
displaying the target parameter layout page through at least two parameter report pages, when the target parameter layout page comprises a number of display line(s) which exceeds the preset number of display line(s); wherein the parameter report area only displays one parameter report page at a time;
when the target parameter layout page comprises the at least two parameter report pages, the interface display method further comprises:
receiving a parameter report page selection instruction for selecting a target parameter report page to display.

17. The interface display method according to claim 16, **characterized in that**, the preset number of display line(s) does not exceed 5.

18. The interface display method according to claim 16, **characterized in that**, further comprising:
receiving a locking instruction from a user for a target parameter on a currently displayed parameter report page, wherein the locking instruction is configured to lock a layout position of the target parameter; and
in response to the locking instruction, locking the layout position of the target parameter, such that, when the target parameter layout page comprises the at least two parameter report pages and the at least two parameter report pages are switched, the layout position of the target parameter remains unchanged in the parameter report area.

19. The interface display method according to claim 18, **characterized in that**, after locking the layout position of the target parameter, the interface display method further comprises:
receiving an unlocking instruction for the target parameter and unlocking the layout position of the target parameter.

20. The interface display method according to claim 1, **characterized in that**, a parameter report area of the monitor comprises a preset number of display column(s), wherein the preset number of display column(s) is less than or equal to 2.

21. A monitor comprising a human-machine interaction module and a processor; **characterized in that**, the human-machine interaction module is configured to receive a measurement mode selection instruction and/or a selection instruction for a functional control element, which are/is inputted by a user, wherein the human-machine interaction module is further configured to display a work interface;
wherein the work interface comprises:
a mode selection area, which is configured to display measurement modes and to receive the measurement mode selection instruction, wherein each measurement mode is associated with a corresponding parameter layout page; the measurement modes are at least configured to characterize a working mode of the monitor, wherein the working mode comprises a continuous measurement working mode and a discontinuous measurement working mode; wherein the mode selection area is further configured to determine multiple first measurement modes according to the continuous measurement working mode, wherein the multiple first measurement modes respectively correspond to multiple first parameter layout pages; wherein the mode selection area is further configured to determine multiple second measurement modes according to the discontinuous measurement working mode, wherein the multiple second measurement modes respectively correspond to multiple second parameter layout pages; wherein the first parameter layout pages are at least partially different from the second parameter layout pages; wherein the measurement mode selection instruction is configured to select a target measurement mode from a measurement mode identifier list, wherein the measurement mode identifier list comprises the multiple first measurement modes and the multiple second measurement modes;
a state display area, which is configured to display state information of the monitor;
a parameter report area, which is configured to display a target parameter layout page which corresponds to the target measurement mode, according to the target measurement mode; wherein the target parameter layout page comprises at least a display area of a measurement parameter and measurement data which is located within said display area; wherein when the target parameter layout page belongs to the first parameter layout pages, the target parameter layout page comprises a first parameter report area; when the target parameter layout page belongs to the second parameter layout pages, the target parameter layout page comprises a second parameter report area, wherein the first parameter report area is at least partially different from the second parameter report area;
a functional control element area, which comprises at least one functional control element;
wherein the processor is configured to execute a processing flow which corresponds to a selected target functional control element, in response to the selection instruction for the functional control element.

22. The monitor according to claim 21, **characterized in that**, the mode selection area is specifically configured to display the measurement mode identifier list according to a measurement situation of the monitor, wherein the measurement mode identifier list comprises identifier(s) of each measurement mode in the measurement situation;
the human-machine interaction module is specifically configured to receive a target identifier, which is selected through the measurement mode identifier list, wherein the target identifier corresponds to the target measurement mode;
the measurement situation comprises hospital working areas, which at least comprise General ward and Emergency department;
when the measurement situation is General Ward, the continuous measurement working mode in the measurement mode identifier list comprises a continuous monitoring mode, the discontinuous measurement working mode in the measurement mode identifier list comprises a general round mode and an initial evaluation mode;
when the measurement situation is Emergency Department, the continuous measurement working mode in the measurement mode identifier list comprises a continuous monitoring mode, the discontinuous measurement working mode in the measurement mode identifier list comprises a triage mode and a spot check mode.

23. The monitor according to claim 21, **characterized in that**, the parameter report area further comprises a parameter measurement indication identifier, which is configured to provide a measurement indication according to a measurement order of at least two measurement parameters, so as to indicate to measure at least one measurement parameter of the at least two measurement parameters.

24. The monitor according to claim 23, **characterized in that**, the parameter measurement indication identifier is further configured to:
distinguishingly display a display area of a measurement parameter which has a measurement priority, and display area(s), which is/(are) not the display area of the measurement parameter which has the measurement priority and is/(are) on the target parameter layout page;
wherein the measurement parameter which has the measurement priority, is a measurement parameter to be measured currently, and is determined from unmeasured measurement parameter(s), according to the measurement order and ranking position(s) of currently measured measurement parameter(s) in the measurement order.

25. The monitor according to claim 23, **characterized in that**, the parameter measurement indication identifier is further configured to:
mark display area(s) which is(are) unfilled with measurement data on the target parameter layout page as unfilled; and/or
when measurement data of a target measurement parameter in the at least one measurement parameter is received and the measurement data of the target measurement parameter is filled into a display area which corresponds to the target measurement parameter, mark the display area of the target measurement parameter as filled.

26. The monitor according to claim 21, **characterized in that**, the display area of the measurement parameter is further configured to:
receive at least one of a measurement mode switching instruction and a display mode change instruction, which are inputted for the measurement parameter, wherein the measurement mode switching instruction is configured to switch from a current measurement mode to a target measurement mode, wherein the display mode change instruction is configured to switch from a current display mode to a target display mode;
switch from the current measurement mode of the measurement parameter in the display area of the measurement parameter to the target measurement mode of the measurement parameter, in response to the measurement mode switching instruction; and
change from the current display mode of the measurement parameter in the display area of the measurement parameter to the target display mode of the measurement parameter, in response to the display mode change instruction.

27. The monitor according to claim 26, **characterized in that**, the display area of the measurement parameter comprises a measurement mode icon which is configured to display a measurement mode of the measurement parameter;
wherein the measurement mode icon is further configured to display at least two measurement modes of the measurement parameter for selecting one measurement mode therefrom as the target measurement mode.

28. The monitor according to claim 26, **characterized in that**, the display area of the measurement parameter comprises at least two area subpages, wherein each area subpage is configured to correspondingly display the measurement parameter according to one display mode; wherein the at least two area subpages switch according to the display mode change instruction, so as to determine the target display mode.

29. The monitor according to any one of claims 21-28, **characterized in that**, the display area of the measurement parameter further comprises a re-measurement control element, which is configured to:
receive an inputted re-measurement instruction and replace current measurement data with new measurement data.

30. The monitor according to any one of claims 21-28, **characterized in that**, the display area of the measurement parameter further comprises an open control element, which is configured to:
open a hidden area of the display area of the measurement parameter, when receiving a display area operation instruction for the measurement parameter, which indicates to open the display area of the measurement parameter; and
hide the hidden area of the display area of the measurement parameter, when receiving a display area operation instruction for the measurement parameter, which indicates to hide the display area of the measurement parameter.

31. The monitor according to claim 30, **characterized in that**, in order to receive the display area operation instruction, which is inputted by the user, the human-machine interaction module is specifically configured to:
receive, through swiping gesture input, the display area operation instruction of the measurement parameter.

32. The monitor according to claim 21, **characterized in that**, the display area of the measurement parameter on a first parameter layout page of the first parameter layout pages, comprises alarm limit information of the measurement parameter.

33. The monitor according to claim 21, **characterized in that**, when the target parameter layout page comprises at least two parameter report pages, the parameter report area is configured to display one parameter report page, and the at least two parameter report pages are switched in the parameter report area.

34. The monitor according to claim 33, **characterized in that**, the display area of the measurement parameter further comprises a locking control element, which is configured to:
receive a locking instruction from the user for a target parameter on a currently displayed parameter report page;
wherein the locking instruction is configured to lock a layout position of the target parameter; such that when the target parameter layout page comprises the at least two parameter report pages and the at least two parameter report pages are switched, the layout position of the target parameter remains unchanged in the parameter report area.

35. The monitor according to claim 34, **characterized in that**, the locking control element is further configured to:
receive an unlocking instruction for the target parameter and unlock the layout position of the target parameter; wherein the layout position of the target parameter changes in the parameter report area, when switching between the at least two parameter report pages.

36. The monitor according to claim 21, **characterized in that**, a first parameter layout page of the first parameter layout pages comprises a first functional control element area, a second parameter layout page of the second parameter layout pages comprises a second functional control element area; wherein first functional control element(s) in the first functional control element area is(are) at least partially different from second functional control element(s) in the second functional control element area.

37. The monitor according to claim 36, **characterized in that**, the first functional control element area comprises at least one of: a review functional control element, a case reference functional control element, a save functional control element, and a clearing functional control element; wherein:
the review functional control element is configured to display, for the user, a historical record of parameter measurement data, which is stored by the monitor,
the case reference functional control element is configured to display, for the user, parameter measurement data of a target patient;
the save functional control element is configured to save the measurement data on the target parameter layout page; and
the clearing functional control element is configured to clear the measurement data on the target parameter layout page.

38. The monitor according to claim 36, **characterized in that**, the second functional control element area comprises an alarm functional control element.

39. The monitor according to claim 38, **characterized in that**, the alarm function control element comprises at least one of an alarm pause functional control element and an alarm reset functional control element.
